# EUROPÄISCHE PATENTSCHRIFT

(11) **EP 1 353 625 B1**
(45) Veröffentlichungstag und Bekanntmachung des Hinweises auf die Patenterteilung: **04.03.2009**
(21) Anmeldenummer: 02716081.1
(22) Anmeldetag: 24.01.2002
(51) Int. Cl.: A61K 6/10, B01J 31/22, C08L 83/04

(54) **ZWEISTUFIG HÄRTBARE MISCHERGÄNGIGE MATERIALIEN**
MATERIALS WHICH CAN BE MIXED AND HARDENED IN TWO STEPS
MATERIAUX POUVANT ETRE MALAXES ET DURCIS EN DEUX ETAPES

(30) Priorität: 25.01.2001 EP 01101713; 17.02.2001 DE 10107519
(43) Veröffentlichungstag der Anmeldung: 22.10.2003
(73) Patentinhaber: Kettenbach GmbH & CO. KG, 35713 Eschenburg (DE)
(72) Erfinder: BUBLEWITZ, Alexander, 35745 Herborn (DE); REBER, Jens-Peter, 58540 Meinerzhagen (DE); NAGEL, Ulrich, 72076 Tübingen (DE)
(74) Vertreter: KEIL & SCHAAFHAUSEN Patentanwälte
(86) Internationale Anmeldenummer: PCT/EP2002/000700
(87) Internationale Veröffentlichungsnummer: WO 2002/058641

(56) Entgegenhaltungen:
- EP-A- 0 891 763
- EP-A- 1 077 226
- WO-A-97/40102
- DE-A- 19 837 855
- US-A- 6 013 711

## Beschreibung

Gegenstand der vorliegenden Erfindung sind Mehrkomponenten-systeme, Komponenten, Mischungen und Verfahren zur Abdrucknahme.

Additionsvernetzende Silicone nutzen die (Edel)metall (A) katalysierte Hydrosilylierung zwischen einem vinylendgestoppten Polydimethylsiloxan (B) und einem Polymethylhydrogensiloxan (C) zum Aufbau eines dreidimensionalen elastomeren Netzwerks. Dabei wird die SiH-Gruppe des Polymethylhydrogensiloxans an die Si-Vinyl-Gruppe unter Bildung einer Ethylenbrücke addiert. Sobald die Komponenten A, B und C in Kontakt gebracht werden, beginnt unmittelbar die Vernetzungsreaktion. Um anwendergerechte Verarbeitungsbedingungen zu schaffen, werden additionsvernetzende Silicone als 2 - Komponenten-Materialien in Form getrennter Komponenten 1 und 2 aufbewahrt und erst kurz vor der Anwendung zusammengemischt.

Die Komponente 1 enthält neben den Vinylorganopolysiloxanen (B), den für die Vernetzungsreaktion notwendigen (Edel)metall - Katalysator (A), (in der Regel Platin-Katalysator). Die Komponente 2 enthält zwingend Polymethylhydrogensiloxan (C) als Vernetzer und gegebenenfalls Vinylorganopolysiloxan.

Zur Einstellung der Verarbeitungszeit bzw. Topfzeit dieser additionsvernetzenden 2 - Komponenten Silicone werden sogenannte Inhibitoren eingesetzt, die in den Katalysezyklus eingreifen und somit die Reaktionsgeschwindigkeit steuern.

Beispiele für solche oben genannte Inhibitoren sind Benzotriazol, Ethinylcyclohexanol, kurzkettige vinylhaltige Organopolysiloxane wie Divinyltetramethyldisiloxan und vinylhaltige cyclische Siloxane wie Tetravinyltetramethylcyclotetrasiloxan, Diethylmaleat und n-Octylsilan (C₈ H₁₇ SiH₃).

Als Inhibitoren werden außerdem, wie z.B. in der DE19837855 A1 oder WO97/40102 kurzkettige Alkinylverbindungen oder Dialkinylverbindungen verwendet, die die Platinkatalysatoren komplexieren oder chelatisieren.

Die über die Hydrosilylierung laufende Vernetzung der additionsvernetzenden Silicone reagiert sehr sensibel auf Katalysatorgifte, wie Stickstoff-, Phosphor- oder Schwefelverbindungen, wie sie in organischen Verbindungen wie z.B. Aminosäuren oder z.B. in Latexhandschuhen vorkommen. Das heißt, die Abbindereaktion wird bei Kontakt mit diesen Katalysatorgiften verzögert und im schlimmsten Fall zum Erliegen gebracht.

Additionsvernetzende Silicone finden in der Technik breiten Einsatz bei der Verwendung als Abformmassen (z.B. Modellbau), Vergussmassen für die Elektro- und Autoindustrie, Dichtmassen für die Bau- und Automobilindustrie (siehe Andreas Tomanek, "Silicone und Technik", 1990, Hanser Verlag München; W. Noll, "Chemie und Technologie der Silicone", 1968, Verlag Chemie; Silicone "Chemie und Technologie", 1989, Vulkan Verlag, Essen).

In der Zahnheilkunde werden additionsvernetzende Silicone für die Zahnabdrucknahme, Bissregistrierung und zur Zahnprothesenunterfütterung erfolgreich eingesetzt.

Bei der Verwendung als Zahnabdruckmaterial können verschiedene Abformtechniken angewendet werden:
- Monophasenabformung (einphasig und einzeitig),
- Sandwichtechnik (zweiphasig und einzeitig),
- Doppelmischtechnik (zweiphasig und einzeitig) und
- Korrekturabformung (zweiphasig und zu zwei verschiedenen Zeiten).

Bei der Sandwich- und Korrekturabformung werden zwei Siliconabformmaterialien mit unterschiedlicher Mischungskonsistenz (ISO 4823) eingesetzt.

Im ersten Schritt wird dabei der Konfektionsabformlöffel mit einem knetbaren Trägermaterial (Putty) beschickt und in einem zweiten Schritt mit einem dünnfließenden Silicon überschichtet. Dies geschieht bei der Sandwichtechnik zeitgleich während der Verarbeitungszeit der beiden Materialien, dann werden beide Materialien gleichzeitig in den Patientenmund appliziert.

Bei der Korrekturabformung wird das knetbare Putty-Material als Vorabdruckmaterial im Patientenmund zur Aushärtung gebracht, nach der Mundentnahme zurückgeschnitten und in einem zweiten nachgeschaltetem Schritt mit dünnfließendem Material überschichtet und wiederum im Patientenmund zur Aushärtung gebracht.

Das Trägermaterial (Putty) wird nach dem Stand der Technik (Dr. Bernd Wöstmann, Habilitationsschrift 1992, "Zum derzeitigen Stand der Abformung in der Zahnheilkunde", Universität Münster; J. Wirz, "Abformungen in der zahnärztlichen Praxis", 1993, Gustav Fischer Verlag) vorwiegend als knetbares Material verwendet. Dies hat den Vorteil, dass bei der Abdrucknahme im Konfektionslöffel ein höherer Staudruck erzeugt wird. Bei der Sandwichtechnik führt dies dazu, dass das dünnfließende Korrekturmaterial in enge Kavitäten und den Sulcus einfließt und somit eine hochpräzise detailgenaue Abformung gewährleistet.

Bei der Vorabdrucknahme des Korrekturabformmaterials hat das knetbare Trägermaterial aufgrund seiner zähen Konsistenz den Vorteil, dass es weniger fein ausfließt und somit mehr Raum für die zeitlich nachgeschalteten Korrekturabformschritte mit dünnfließendem Material bereithält.

Um zusätzlich Raum für das im nachgeschaltetem Schritt eingesetzte Korrekturmaterial zu schaffen, kann es notwendig sein, dass ausgehärtete Trägermaterial auszuschneiden, d.h. in der Vorabformung werden die abgeformten Zahnzwischenräume weggeschnitten und Abflusskanäle gelegt.

Aus diesem Grund muss das ausgehärtete Trägermaterial eine gute Beschneidbarkeit z.B. mit einem Skalpell aufweisen.

Ansonsten würde es in diesem Schritt Verpressungen des Trägermaterials hervorrufen (Prof. Dr. Wöstmann, Uni Gießen: "Zum derzeitigen Stand der Abformung in der Zahnheilkunde", Münster 1992).

Das für die oben genannte Abformtechniken verwendete Trägermaterial (Putty) hat für den Anwender aber folgende Nachteile:

Das Dosieren erfolgt mit Dosierlöffein mit denen das knetbare Material aus den Vorratsdosen mit relativ hohem Kraftaufwand umständlich entnommen wird. Anschließend werden die unterschiedlich eingefärbten Komponenten mit einer Mischgabel oder mit der Hand zu einer homogenen Masse verknetet. Auch dieser Vorgang ist mehr oder weniger umständlich und bei Verwendung einer Knetgabel mit einem hohen Kraftaufwand durchzuführen. Aus dieser Art des Dosierens und Mischens können sich folgende Fehler einschleichen:
- es besteht die Gefahr der Fehldosierung und
- es besteht das Risiko einer nicht homogenen Vermischung der beiden Komponenten.

Beide Faktoren führen zu einer unzureichenden Vernetzung, so dass die Präzision bei der Modellherstellung nicht mehr gegeben ist.

Beim Dosieren von knetbaren Vorabformmaterialien (Putties) besteht die Gefahr der Kontamination der Komponenten mit der jeweils anderen Komponente. Erfahrungsgemäß lassen sich trotz Farbcodierung Anwendungsfehler nicht vermeiden.

Häufig werden die Dosierlöffel oder die Deckel der Gebinde von Komponente 1 und 2 vom Anwender verwechselt oder es wird mit kontaminierten Fingern bzw. Handschuhen in die Vorratsdose gegriffen. Auf diese Weise kontaminierte Abformmassen sind für den weiteren Einsatz unbrauchbar; sie weisen z.B. abgebundene Bereiche in Form von Klümpchen auf.

Beim üblichen Mischen von knetbaren Vorabformmaterialien (Putties) von Hand, besteht die Gefahr, dass aus der Haut des Anwenders oder aus dessen Schutzhandschuhen Katalysatorgifte herausgelöst werden, die zu einer Abbindeverzögerung oder im schlimmsten Fall zu einer Verhinderung der Abbindung des Abformmaterials führen können.

Im Markt sind sogenannte Putty-Cartridge-Materialien (z.B. Reprosil Quixx Putty, Fa. Dentsply Caulk) bekannt, die sich mit den handelsüblichen Misch- und Dosiersystemen verarbeiten lassen und die alternativ zu knetbaren Vorabformmaterialien eingesetzt werden können. Es handelt sich um dünn- bis zähfließende Materialien nach ISO 4823, die aufgrund ihrer schnellen Vernetzungscharakteristik schon während der Verarbeitungszeit einen relativ hohen Vernetzungsgrad zeigen. Dies führt dazu, dass die ursprünglich dünn- bis zähviskose Anfangskonsistenz sich während der Verarbeitungszeit stark erhöht und dass bei der Abdrucknahme dem Anwender das Gefühl eines Puttymaterials vermittelt wird (hoher Anpressdruck).

Diese Abformmassen haben den Nachteil, dass die Gesamtverarbeitungszeit für den Zahnarzt stark eingeschränkt ist. Bei Überschreitung der vorgegebenen Verarbeitungszeiten oder bei leicht erhöhten Umgebungstemperaturen besteht die Gefahr, dass die Abformmasse bereits hohe elastische Anteile ausgebildet hat oder im Extremfall schon ausgehärtet ist, so dass die Abformungen durch endogene Spannungen, Rückstellkräfte und Verpressungen stark verfälscht und mithin unbrauchbar sind.

Aufgabe der vorliegenden Erfindung ist es, mischergängige Abformmaterialien auf Basis von additionsvernetzenden Polydimethylsiloxanen herzustellen, die sich aufgrund ihrer in der Anfangsphase dünn- bis zähviskosen Mischungskonsistenz aus den in jüngster Zeit entwickelten automatischen Misch- und Dosiersystemen austragen lassen und aufgrund ihres zweistufigen Reaktionsmechanismus in einem ersten Reaktionsschritt während und nach der Mischzeit in eine zweite Mischungskonsistenz mit erhöhter Viskosität übergehen.

In einem zweiten Reaktionsschritt nach einer einstellbaren Verarbeitungszeit, während der ein Abdruck genommen wird, soll das Abformmaterial vollständig zu einem elastischen Endzustand aushärten, in dem ein Abformergebnis festgehalten wird.

Unter mischergängiger Abdruckmasse ist eine Mehrkomponenten Abdruckmasse zu verstehen, die z.B. aus einer 2-Komponenten- Einweg-Kartusche über einen statischen Mischer z.B. von Fa. Mixpac (Keller EP 0 615 787 A1, EP 0 730 913 A1) oder aus Schlauchbeutelfolien in Doppelkammermehrwegkartuschen mittels dynamischem Mischer z.B. im "Mixstar"-Gerät der Fa. DMG-Mühlbauer (PCT/EP 98/01993 und PCT /EP 98/01858) oder im "Pentamix I" bzw. "Pentamix II" Gerät der Fa. Espe (EP-A-0492413 und EP-A-0492412) austragbar ist.

Die Anforderung der Mischergängigkeit kann von Abformmaterialien erfüllt werden, die zu Beginn der Mischzeit nach ISO 4823 im Konsistenzbereich von größer 26 mm, insbesondere größer 30 mm liegen.

Das der Erfindung zugrunde liegende technische Problem darin, eine Abformmasse zu schaffen, die die oben aufgezeigten Nachteile vermeidet und ein Verfahren bereitzustellen, das in einfacher und zuverlässiger Weise die Abformung abzuformender Gegenstände erlaubt. Insbesondere lag das Problem vor, ein Mehrkomponentenabformmaterial bereitzustellen, das sich einfach und gut mischen lässt und mischergängig ist, das sich zur Abdrucknahme eignet und in einen elastischen festen Zustand übergeht.

Diese Probleme werden überraschenderweise gelöst durch Bereitstellung eines Mehrkomponentensystem zur Abdrucknahme, das
(a) mindestens eine Verbindung mit mindestens zwei Alkenylgruppen,
(b) mindestens ein Organohydrogenpolysiloxan und
(c) mindestens einen Hydrosilylierungskatalysator enthält,
   dadurch gekennzeichnet, dass
(d1) mindestens eine polymere Verbindung mit mindestens einer Alkinylgruppe und/oder
(d2) mindestens eine Verbindung mit mindestens einer Si - OR - Struktureinheit, wobei R = H, Alkyl, Alkoxyalkyl oder Acyl ist, und
   wenn eine Verbindung (d2) mit mindestens einer Si - OR - Struktureinheit enthalten ist,
(e) mindestens einen Kondensationskatalysator und/oder -vernetzer enthalten ist.

Bevorzugt ist dabei die Bereitstellung eines Mehrkomponentensystem zur Abdrucknahme mit mindestens zwei Komponenten A und B, wobei die Komponente A
(a) mindestens eine Verbindung mit mindestens zwei Alkenylgruppen und
(b) mindestens ein Organohydrogenpolysiloxan,
   sowie
(d1) mindestens eine polymere Verbindung mit mindestens einer Alkinylgruppe und/oder
(d2) mindestens eine Verbindung mit mindestens einer Si - OR - Struktureinheit, wobei R = H, Alkyl, Alkoxyalkyl oder Acyl ist, enthält,
   die Komponente B
(c) mindestens einen Hydrosilylierungskatalysator und
   die Komponente A und/oder B, wenn eine Verbindung (d2) mit mindestens einer Si - OR - Struktureinheit enthalten ist,
(e) mindestens einen Kondensationskatalysator und/oder -vernetzer enthält.

Die Verbindungen (a), (b) und (d1) sind Einzelverbindungen.

Die erfindungsgemäße Komponente A und/oder B kann zusätzlich
(f) Inhibitoren der Kondensationsreaktionen von Kondensationskatalysatoren und/oder -vernetzern mit Si - OR - Struktureinheiten enthaltenden Verbindungen, wobei R = H, Alkyl, Alkoxyalkyl oder Acyl ist,
(g) wasserabgebende Mittel
(h) Trockenmittel
(i) inerte Trägerstoffe,
(j) Verbindungen zur Reaktionsinhibierung der Hydrosilylierungsreaktion,
(k) verstärkende Füllstoffe,
(l) nicht verstärkende Füllstoffe und/oder
(m) Hilfsstoffe enthalten.

Die der Erfindung zugrundelegende Aufgabe wird durch Ausnutzung eines zweistufigen Reaktionsmechanismus gelöst, der während und nach dem Mischen der Komponenten A und B einsetzt und der die unterschiedliche Reaktivität bei der Additionsvernetzung der Alkenyl- und Alkinylgruppen gegenüber SiH-Gruppen ausnutzt und/oder die unterschiedlichen Reaktionsmechanismen bei der Additionsvernetzung zwischen Alkenyl- und SiH - Gruppen gegenüber der Kondensationsvernetzung von Si - OR - Gruppen, wobei R = H, Alkyl, Alkoxyalkyl oder Acyl ist, mit Kondensationskatalysatoren ausnutzt.

Überraschenderweise zeigt sich bei der Kombination der Hydrosilylierungsreaktion von Si Vinyl- und Si Ethinyl-Gruppen, dass
a) die Reaktion zeitlich nacheinander abläuft und somit zur Verwendung in zweistufig härtbaren Abformmaterialien geeignet ist;
b) die Reaktanden in einem Mehrkomponenten- insbesondere 2-Komponentenmaterial so auf die Einzelkomponenten aufgeteilt werden können, z.B. Doppelkammerkartusche oder Folienschlauchbeutel, dass diese über einen Zeitraum von mindestens 12 Monaten bei Raumtemperatur lagerstabil sind, d.h. keine Reaktivitätsverluste aufweisen.

Überraschenderweise zeigt sich bei der Kombination der Kondensationsreaktion (Hydroxypolysiloxan mit Kondensationskatalysatoren wie z.B. Titanorthoester) mit der Hydrosilylierungsreaktion (Si Vinyl und/oder Si Ethinyl mit Organohydrogenpoiysitoxanen in Gegenwart von Pt-Katalysatoren), dass
a) die Reaktionen zeitlich nacheinander ablaufen, bei Einsatz geeigneter Inhibitoren;
b) die Reaktionen unabhängig voneinander ablaufen, ohne dass sich die Reaktanden negativ auf die Reaktivität der jeweils anderen Reaktionen auswirken;
c) die Reaktanden in einem Mehrkomponenten- insbesondere 2-Komponentenmaterial so auf die Einzelkomponenten aufgeteilt werden können, z.B. Doppelkammer-Kartusche oder Folienschlauchbeutel, dass diese über einen Zeitraum von mindestens 12 Monaten bei Raumtemperatur lagerstabil sind, d.h. keine Reaktivitätsverluste und Reduzierung der mechanischen Eigenschaften im Vulkanisat aufweisen;
d) der Konzentrationsbereich der eingesetzten Metallalkoxide so gewählt werden kann, dass im Praxisbetrieb, d.h. im Einsatz in Kartuschen und Schlauchbeuteln keine Verstopfung der Austrittskanäle durch Luftfeuchtigkeitszutritt bedingte "Verkieselung" auftritt.

Dabei werden die Komponenten vorzugsweise so ausgewählt, dass die Kondensations- und die Hydrosilylierungsreaktion bei 10 bis 40°C stattfinden, so dass die Reaktionen insbesondere bei Mund- und Raumtemperatur durchgeführt werden können.

Die erfindungsgemäß verwendeten Alkinylverbindungen (d1) sind polymere (langkettige) Verbindungen. Der Grund dafür ist, dass bei einer relativ schnell stattfindenden ersten Reaktion, in der die Alkinylverbindung mit Si-H Verbindungen reagiert, die Viskosität der Mischung deutlich zunehmen soll. Dies wird nur erreicht, wenn die Alkinylverbindung eine gewisse Kettenlänge aufweist. "Polymere im Sinne der Erfindung bedeutet daher: Wenn die Alkinylverbindung im wesentlichen ein Polysiloxan ist, sind zum Beispiel Verbindungen mit mindestens 20, bevorzugt 50, besonders bevorzugt 500 Si-O- Einheiten verwendbar. Wenn die Alkinylverbindung im wesentlichen eine Kohlenwasserstoff-Verbindung ist, sind zum Beispiel solche mit mindestens 15, vorzugsweise 20, insbesondere 25 C-Atomen bevorzugt. Allgemein erhöhen Kohlenwasserstoffverbindungen die Viskosität stärker als Polysiloxanverbindungen vergleichbarer Länge. Der Anteil der Alkinylverbindungen an der Abformmasse beträgt vorzugsweise mindestens 1%, besonders bevorzugt mindestens 2 oder 5%.

Die Konzentration der Alkinylverbindung und die Kettenlänge der Alkinylverbindung stehen bei der Auswahl der Mischung in reziprokem Zusammenhang. Je länger die Kettenlänge ist, desto niedriger muss die Konzentration der Alkinylverbindung gewählt werden. Umgekehrt kann der gewünschte Viskositätsanstieg erreicht werden, wenn eine relativ hohe Konzentration einer polymeren Alkinylverbindung mit relativ kurzer Kettenlänge gewählt wird.

Nicht erfindungsgemäß einsetzbar sind in diesem Zusammenhang kurzkettige Dialkinylverbindungen, die nach dem Stand der Technik (z.B. DE 19837855 A1) als Inhibitoren zur Chelatisierung von Pt oder anderen Metallen verwendet werden. Bevorzugte Kettenlängen solcher Inhibitoren sind C₆ bis C₈, da nur dann ein guter Chelatisierungseffekt erzielt wird. Aufgrund der kurzen Kettenlängen könnten solche Inhibitoren keinen signifikanten Viskositätsanstieg bewirken.

In bevorzugten Ausführungsformen ist die Alkinylverbindung (d1) der Komponente A

R¹-C ≡ C-X-R²

und die Si - OR -Verbindung nach (d2): 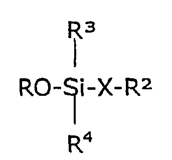 mit R = H, Alkyl, Alkoxyalkyl oder Acyl;
- R¹ =: Alkyl-, Aryl-, Arylalkyl, halogensubstituierte Alkyl- und Aryl- gruppen, Cyanalkyl-, Cycloalkyl-, Cycloalkenyl-, insbesondere H-, -OH und -OR, sowie Kombinationen davon; 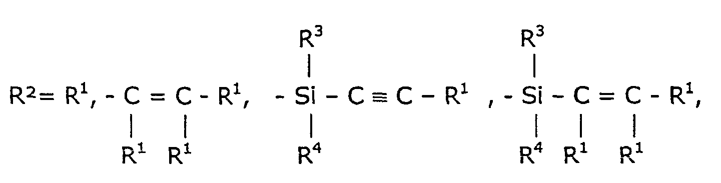 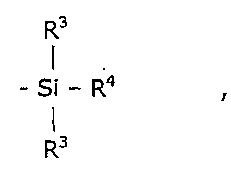 - C ≡ C - R¹,
R³ = Alkenyl-, Alkinyl, Halogen-, Aryl-, Alkylaryl-, H-, Halogensubstitu- ierte Alkyl- und Arylgruppen insbesondere Alkyl-, Alkoxy- und Hy- droxyl, sowie Kombinationen davon,
R⁴ = R³, oder R⁴ verschieden von R³, wobei R⁴ insbesondere Alkoxy-, Hydroxyl -, Alkyl-, Methyl-, Alkinyl-, Ethinyl- oder Kombinationen davon; und
- X =: Polysiloxan, Oligokieselsäureester, Polykieselsäureester, Polyether, polymere Kohlenwasserstoffe, Polyester und Copolymere der oben genannten Verbindungen ist.

In den Polymeren können polymere Seitenketten und/oder die Reste R¹ und/oder R² vorhanden sein. Die Kette kann Alkenyl- oder Alkinylgruppen enthalten.

Verbindungen, bei denen R = Alkyl, Alkoxyalkyl oder Acyl ist werden bevorzugt eingesetzt, wenn Sn-Katalysatoren enthalten sind.

In der Komponente A können als Verbindungen nach (d1) oder (d2) insbesondere solche mit der Strukturformel 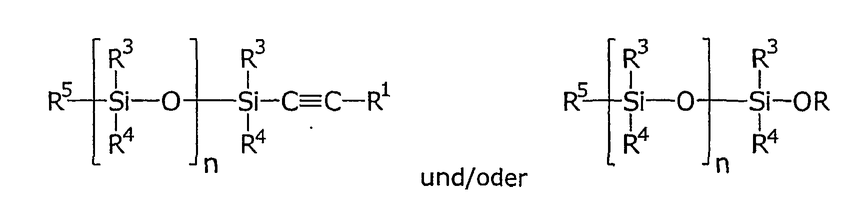 mit n = 7 - 6000, bevorzugt 20 - 6000, insbesondere 100 - 6000,
mit R und R¹ bis R⁴ wie bereits oben aufgeführt und
mit R⁵ = -C ≡ C-R¹, H-, Alkyl-, Aryl-, 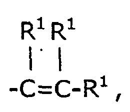 Alkylaryl-, Halogen, -OH halogensubstituierte Alkyl- und Arylgruppen, - OR, Aminoalkyl-, Epoxy-, Cyanalkyl-, Cycloalkyl-, Alkylhydroxyl-, Methacrylat-, Acrylat-, Mercaptoalkyl-, Carboxylat-, Carboxyalkyl- oder Succinicanhydrid
enthalten sein.

Solche Verbindungen können nach bekannten Verfahren aus EP0639622 B1, EP0959095 A2, EP0926206A2, DE19822679 A1 und DE19757308 A1 hergestellt werden.

### Bei

Bevorzugte Alkenylverbindung (a) der Komponente A sind solche mit der Struktur 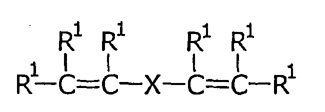 oder 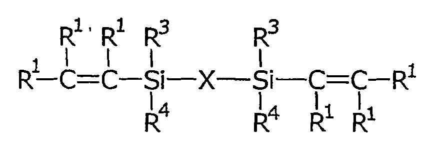 oder 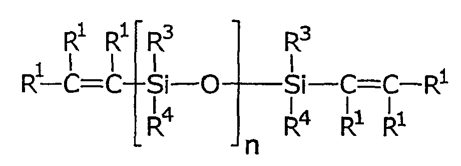 mit n = 0 - 6000,
wobei die Reste R¹, R², R³, R⁴ und X wie, bereits oben aufgeführt ausgewählt sind.

Die bevorzugte Alkenylverbindung (a) kann auch ein Silan-Dendrimer mit terminalen Alkenylgruppen sein.

Bevorzugte Organohydrogenpolysiloxane (b), die in der Komponente A enthalten sind, sind Polyalkyl-, Polyaryl- und Polyalkylaryl-, Polyhalogenalkyl-, Polyhalogenaryl- oder Polyhalogenalkylaryl-Siloxane. Sie können als Oligomere oder Polymere in linearer, verzweigter oder zyklischer Form oder als QM-Harz vorliegen und weisen mindestens eine Si-H-Bindung auf.

Als Kondensationskatalysatoren und/oder -vernetzer (e) werden in der Komponente B vorzugsweise Aluminiumalkoxide, Antimonalkoxide, Bariumalkoxide, Boralkoxide, Calciumalkoxide, Ceralkoxide, Erbiumalkoxide, Galliumalkoxide, Siliciumalkoxide, Germaniumalkoxide, Hafniumalkoxide, Indiumalkoxide, Eisenalkoxide, Lanthaniumalkoxide, Magnesiumalkoxide, Neodyniumalkoxide, Samariumalkoxide, Strontiumalkoxide, Tantalalkoxide, Titanalkoxide, Zinnalkoxide, Vanadiumalkoxidoxide, Yttriumalkoxide, Zinkalkoxide, Zirconiumalkoxide, Titan- oder Zirkoniumverbindungen, insbesondere Titan- und Zirconiumalkoxide, sowie Chelate und Oligo- und Polykondensate der o.g. Alkoxide, Dialkylzinndiacetat, Zinn(II)octoat, Dialkylzinndiacylat, Dialkylzinnoxid und Doppelmetallalkoxide eingesetzt. Doppelmetallalkoxide sind Alkoxide, die zwei verschiedene Metalle in einem bestimmten Verhältnis enthalten. Insbesondere werden eingesetzt: Titantetraethylat, Titantetrapropylat, Titantetraisopropylat, Titantetrabutylat, Titantetraisooctylat, Titaniso-propylattristeaorylat, Titantriisopropylatsteaorylat, Titandiisopropylat-disteaorylat, Zirkoniumtetrapropylat, Zirkoniumtetraisopropylat, Zirkoniumtetrabutylat. Außerdem sind Titanate, Zirkonate und Hafnate wie in DE4427528 C2 und EP0639622 B1 beschrieben verwendbar.

Als Hydrosilylierungskatalysatoren (c) werden in Komponente B vorzugsweise Übergangsmetalle der 8. Nebengruppe, insbesondere Platin, Palladium und Rhodium oder deren Salze, Komplexe und Kolloide, vorzugsweise Platinkomplexe und Salze der Hexachloroplatinsäüre, insbesondere Platin(0)-1,3-divinyl-1,1,3,3 tetramethyldisiloxan-Komplex, eingesetzt werden.

Als Inhibitoren der Kondensationsreaktionen (f) werden in Komponente A bevorzugt Di-, Tri-, Oligo- und Polydialkylsiloxane der allgemeinen Formel

Z - SiR₂ - O - (SiR₂O)ₙ - SiR₃

oder

Z - SiR₂ - O - (SiR₂O)ₙ - SiR₂ - Z

eingesetzt, wobei Z = OH oder NR₂, R gleiche oder verschiedene gegebenenfalls substituierte Kohlenwasserstoffreste wie Alkyl-, Alkenyl-, Aryl- oder Alkinyl- sind und n= 0 oder eine ganze Zahl von 1 bis 100 ist.

Weiterhin können aliphatische Diole, Diamine, Diphosphane, Polyamine, Polyphosphane oder Polyole, OH-, NH- oder PR- funktionelle Polyether oder andere chelatisierende Verbindungen eingesetzt werden.

Als wasserabgebende Mittel (g) werden in Komponente A vorzugsweise anorganische Füllstoffe, die eine oberflächlich gebundene Restfeuchtigkeit oder im Kristallgitter gebundenes Wasser enthalten, Zeolithe oder gezielt befeuchtete Füllstoffe oder organische Stoffe mit definiertem Wassergehalt eingesetzt.

Bevorzugte Trockenmittel (h) in Komponente A und/oder B sind Zeolithe, getrocknete Füllstoffe oder wasseraufnehmende organische Verbindungen wie Oxazolidine und Alkalisalze der Poly(meth)acrylsäure (Superabsorber).

Als inerte Trägerstoffe (i) werden bevorzugt Mineralöle, verzweigte Kohlenwasserstoffe, Vaseline, Ester, Phtalsäureester, Acetyltributytcitrat, Polyalkylenoxide und Polyester und deren Copolymere verwendet.

Als Verbindungen zur Reaktionsinhibierung der Hydrosilylierungsreaktion (j) werden vorzugsweise kurzkettige Organopolysiloxane der allgemeinen Formel

CH₂ = CH - SiR₂O- (SiR₂O)ₙ - SiR₂ - CH = CH₂ ,

wobei R gleiche oder verschiedene, gegebenenfalls substituierte Kohlenwasserstoffreste wie Alkyl-, Alkenyl-, Aryl-, Alkinyl-, Alkenyl-, sowie Alkinyl-terminierte Siloxanreste sind, und
n = 0 oder eine ganze Zahl von 1 bis 6 ist, eingesetzt.

Es können auch vinylhaltige, cyclische Siloxane, wie beispielweise Tetravinyltetramethylcyclotetrasiloxan oder endständige Doppel- oder Dreifachbindungen enthaltende, organische Hydroxyl-Verbindungen, Diethylmaleat, Alkylsilan, Arylsilan, Alkenylsilan, Alkinylsilan, Benzotriazol, Verbindungen mit 1,4-Enin-Struktureinheit, Verbindungen mit 1,3-Enin-Struktureinheit wie 2-Methyl-1-hexen-3-yne, Ethyl-3-(trimethylsilyl)propynoat, Bis-(phenylethinyl)-dimethylsilan, Diyne wie Decadiyne oder Dodecadiyne, Polyyne, Diene, Polyene wie Decatrien, (1,3-Dioxan-2-yl-ethinyl)trialkylsilan, 1,4-Divinyltetramethyldisilyl-ethan, Amine oder Phosphane eingesetzt werden.

Als verstärkende Füllstoffe (k) werden in der Komponente A und/ oder B bevorzugt hochdisperse, aktive Füllstoffe wie Titandioxid, Aluminiumoxid, Zinkoxid, vorzugsweise naßgefällte oder pyrogen gewonnene Kieselsäure verwendet werden, die hydrophil oder hydrophobiert vorliegen können; oder mineralische, faserförmige Füllstoffe wie Wollastonit oder synthetische, faserförmige Füllstoffe wie Glasfasern, Keramikfasern oder Kunststofffasern eingesetzt. Die BET-Oberfläche dieser Stoffe ist vorzugsweise > 50 m²/g.

Als nicht verstärkende Füllstoffe (I) werden in Komponente A und/oder B bevorzugt Metalloxide, Metalloxidhydroxide, Mischoxide oder Mischhydroxide, vorzugsweise Siliciumdioxid, insbesondere in Form von Quarz und seinen kristallinen Modifikationen, Quarzgut sowie Aluminiumoxid, Calciumoxid, Aluminiumhydroxid, sowie Calciumcarbonat, Kieselgur, Diatomeenerde, Talkum, gemahlene Gläser und Füllstoffe auf Kunststoff-Basis, beispielweise Polymethylmethacrylat, Polycarbonat, Polyvinylchorid, Siliconharzpulver oder Pulver auf Basis fluororganischer Verbindungen eingesetzt, wobei die nicht verstärkenden Füllstoffe auch oberflächenbehandelt (gecoated) eingesetzt werden. Die Oberflächenbehandlung kann z.B. mit Silanen und Fettsäuren erfolgen, die funktionelle Gruppen (z.B. Vinyl-, Allyl-, -SiH) aufweisen können. Geeignet als nicht verstärkende Füllstoffe sind auch organische oder anorganische Hohlkugeln, Massivkugeln und Fasern. Es können auch volle oder hohle Kunststoffteilchen, z.B. auch in sphärischer Form, auf deren Oberfläche anorganische Füllstoffpartikel eingebettet sind, eingesetzt werden. Vorzugsweise ist die BET-Oberfläche der nicht verstärkenden Füllstoffe < 50 m²/g.

Die Hilfsstoffe (m), die in Komponente A und/oder B enthalten sein können, sind vorzugsweise Farbstoffe, Tenside, Opaker, Mattierungsmittel, wie z.B. Titandioxid oder Zinkoxid, Weichmacher, Wasserstoff-Adsorber/Absorber, radioopake Substanzen oder siliziumorganische MQ-Harze mit Si-Vinyl, Si-OH, Si-OR, Si-Ethinyl- und/oder SiH-Gruppen

Das Abformmaterial kann hydrophile Eigenschaften aufweisen, z.B. wenn als Hilfsstoff Tenside zugesetzt werden oder wenn Polyethergruppen enthalten sind.

In Komponente A und/oder B können auch Stoffe zur Einstellung des pH-Bereichs enthalten sein. Dazu zählen bevorzugt Essigsäure, Zitronensäure, Acetyltributylcitrat, Ascorbinsäure, saure verstärkende oder nicht verstärkende Füllstoffe, saure Puffersysteme wie Essigsäure/Natriumacetatpuffer oder Zitronensäure/Citrat-Puffer sowie alkalische verstärkende oder nicht verstärkende Füllstoffe wie z.B. Aluminiumhydroxid, alkalische Puffersysteme wie z.B. Carbonat/Hydrogencarbonat-Puffer, oder alkalische oder saure Ionenaustauscherharze.

Die Erfindung betrifft auch eine Mischung aus den Komponenten. Die Erfindungsgemäße Mischung geht nach dem Mischen der Komponenten in einer ersten Stufe von einer dünneren mischergängigen Ausgangskonsistenz zu einer zäheren, plastischen Phase über, in der das Material bei der Zahnabformung im Abformlöffel einen hohen Stempeldruck aufbaut und in einer zweiten Stufe zu seiner endgültigen elastischen Form aushärtet.

Bevorzugte erfindungsgemäße Mischungen weisen zu Beginn des Mischens in einem ersten Zustand eine mischergängige Konsistenz von > 26 mm, bevorzugt > 30 mm (nach ISO 4823) auf, wonach die Mischung durch Kondensationsreaktionen von SiOR-Gruppen und/oder durch Hydrosilylierungsreaktionen von Alkinylgruppen mit SiH-Gruppen in einen zäheren zweiten Zustand mit einer Konsistenz von < 35 mm, bevorzugt < 30 mm (nach ISO 4823) übergehen. Diese Konsistenz bleibt über einen Zeitraum von mindestens 15 sec, vorzugsweise mindestens zwei Minuten, erhalten. Die Mischungen gehen danach durch eine Hydrosilylierungsreaktion von Alkenylgruppen mit SiH-Gruppen nach Aushärten in einen dritten festen, elastischen Zustand über. Die oben genannte Reaktionsfolge kann auch in umgekehrter Reihenfolge durchlaufen werden.

Für die erfindungsgemäßen zweistufig härtbaren Materialien bedeutet dies, dass die erste Reaktionsstufe, das heißt, der Übergang von der mischergängigen Konsistenz von > 26 mm, insbesondere > 30 mm, in eine zähere Konsistenz von < 35, mm, insbesondere < 30 mm zeitlich so ausgesteuert werden kann, dass die Konsistenzerniedrigung (Viskositätsanstieg) während und nach der Mischzeit gemäß ISO 4823 erfolgt. Das erfindungsgemäße Material wird dabei vorteilhafterweise direkt nach dem Mischen ausgetragen, wenn die erste Reaktion noch nicht oder erst in geringem Masse abgelaufen ist. Auf diese Weise werden zum Beispiel statische oder dynamische Mischer von manuellen Misch- oder Dosiersystemen nicht mit z.B. knetbarer Abformmasse verstopft. Erst nach Austritt aus dem Mischer tritt dann die deutliche Viskositätserhöhung durch die erste Reaktionsstufe des zweistufigen Reaktionsmechanismus ein. Wenn das erfindungsgemäße Material jedoch so gewählt ist, dass das Material höherer Viskosität nach Ablauf der ersten Reaktionsstufe noch austragbar ist, kann die Austragung auch während oder nach dem Ablauf der ersten Reaktionsstufe erfolgen.

Vorzugsweise weisen die beiden einzelnen Komponenten A und B Konsistenzen von >26 mm, insbesondere >30 mm auf. Dadurch wird erreicht, dass die Mischung der beiden Komponenten unmittelbar nach Mischbeginn, das heißt, wenn die erste Reaktion noch nicht oder nur unwesentlich angelaufen ist, eine Konsistenz von >26 mm, insbesondere >30 mm aufweist. Andererseits kann auch eine der beiden Komponenten eine niedrigere Konsistenz und die andere Komponente eine höhere Konsistenz aufweisen, wenn beide Komponenten so aufeinander abgestimmt sind, dass unmittelbar nach Mischbeginn eine Konsistenz der Mischung von >26 mm, insbesondere >30 mm vorliegt.

Die Erfindung umfasst die Mehrkomponentengemische nach dem Mischen, in allen beschriebenen Zuständen und nach dem Aushärten. Die Mischung im dritten, d.h. im ausgehärteten Zustand erfüllt vorzugsweise die Anforderungen, die nach ISO 4823 an ein elastomeres Abformmaterial im ausgehärteten Zustand gestellt werden, wie zum Beispiel die Rückstellung nach Verformung.

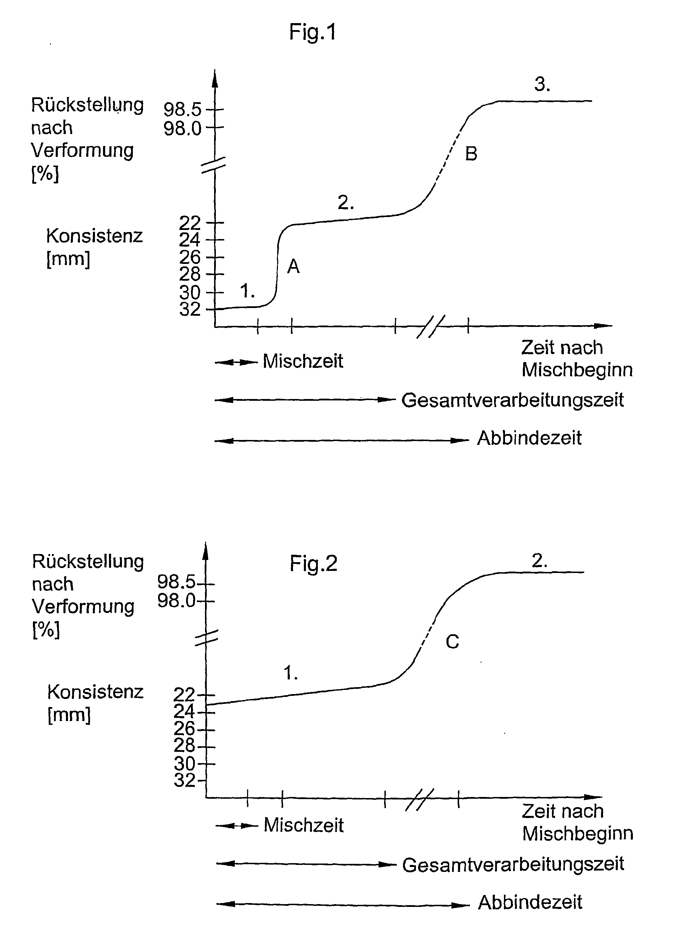

Die Änderung der Konsistenz eines erfindungsgemäßen Mehrkomponentengemischs wird beispielhaft in Fig.1 in idealisierter Form veranschaulicht:

In Fig.1 wird die Konsistenz in mm nach ISO 4823 gegen die Zeit nach dem Mischen der Komponenten A und B aufgetragen. Anhand der Kinetik können deutlich zwei Reaktionsschritte A und B und drei Konsistenzzustände, die über einen gewissen Zeitraum relativ stabil sind, unterschieden werden:
1) 1. Zustand, mischergängige Konsistenz zu Beginn des Mischvorgangs
2) 2. Zustand, heavy bodied oder putty Konsistenz bis zum Ende der Gesamtverarbeitungszeit nach ISO 4823. In diesem 2. Zustand bleibt die Konsistenz über einen gewissen Zeitraum nahezu unverändert. In Folge von Restreaktionen wird unter Umständen ein geringfügiger Anstieg der Viskosität beobachtet.
3) 3. Zustand, ausgehärteter, fester, elastischer Zustand nach Abbindezeit nach ISO 4823
A) Übergang von der mischergängigen in die heavy bodied bis putty consistency durch Kondensationsreaktion der SiOR-Gruppen und/oder Hydrosilylierungsreaktion zwischen Alkinyl- und SiH-Gruppen
B) Übergang von der heavy bodied bis putty consistency durch Vernetzung in den ausgehärteten Zustand durch Hydrosilylierungsreaktion zwischen Alkenyl- und SiH-Gruppen.

In Fig.2 wird dagegen eine entsprechende Kinetik für Konsistenzänderung eines Puttymaterials nach dem Stand der Technik in idealisierter Form gezeigt. Hier können nur zwei zeitweise relativ stabile Konsistenzen unterschieden werden, die durch einen einzigen Reaktionsschritt verknüpft sind:
1) 1. Zustand, nicht mischergängige Konsistenz während der Gesamtverarbeitungszeit nach ISO 4823
2). 2. Zustand, ausgehärteter, fester, elastischer Zustand nach Abbindezeit gemäß ISO 4823
C) Übergang von der ersten, nicht mischergängigen Konsistenz durch Vernetzung in den ausgehärteten Zustand durch Hydrosilylierungsreaktion zwischen Alkenyl- und SiH-Gruppen.

Die Erfindung betrifft auch Verfahren zur Herstellung von Abdrücken von abzuformenden Gegenständen.

Beim erfindungsgemäßen Verfahren wird zunächst eine Abformmasse durch Mischen der Komponenten A und B hergestellt. Die Abformmasse wird zunächst aus einem Behälter über einen Mischer in einem ersten Zustand ausgetragen. Sie geht während und nach dem Mischen in einen zweiten Zustand über, in dem die Viskosität der Abformmasse deutlich erhöht ist, wonach ein Abdruck von einem abzuformenden Gegenstand genommen wird. Die Abformmasse geht danach in einen dritten, festen Zustand über, in dem ein Abformergebnis festgehalten ist, wobei der zweite Zustand durch abgestufte Hydrosilylierungsreaktionen zwischen Alkinyl- und Alkenyl-Struktureinheiten mit Si-H Gruppen enthaltenden Verbindungen und/oder durch abgestufte Additionsreaktion (zwischen Alkenyl und SiH-Gruppen) und Kondensationsreaktion (von SiOR- Gruppen mit Kondensationskatalysatoren) eingestellt wird.

Vorzugsweise ist die Konsistenz der Abformmasse zu Beginn des Mischens im ersten Zustand > 26mm, bevorzugt > 30 mm nach ISO 4823. Die Abformmasse ist in diesem ersten Zustand mischergängig. Die Konsistenz der Abformmasse im zweiten Zustand ist insbesondere < 35 mm, insbesondere < 30 mm (nach ISO 4823), wobei die Abformmasse zäher (viskoser) ist als im ersten Zustand. Bevorzugt verbleibt die Abformmasse in diesem zweiten Zustand bis zum Ende der einstellbaren Gesamtverarbeitungszeit mindestens 15 sec. Vorzugsweise ist der dritte, feste Zustand ein elastischer Zustand.

Mit der vorliegenden Erfindung ist es möglich, Trägermaterialien auf Basis von additionsvernetzenden Polymeren, insbesondere Polydimethylsiloxanen, für die Zahnabformung herzustellen, die sich aufgrund ihrer mischergängigen Konsistenz aus den in jüngster Zeit entwickelten automatischen Misch- und Dosiersystemen austragen lassen.

Diese heavy bodied bis putty consistency bleibt über einen einstellbaren Zeitraum, insbesondere der vom Zahnarzt benötigten Verarbeitungszeit, erhalten.

Gegen Ende der Verarbeitungszeit wird das Abformmaterial in den Mund eingebracht. Nun setzt die zweite Reaktionsstufe ein und das Material härtet vollständig aus. Das erhaltene Vulkanisat weist die mechanischen Eigenschaften einer ausgehärteten Dentalabformmasse nach ISO 4823 auf.

Auf diese Weise werden die Erfordernisse und Vorteile eines heavy bodied bis putty consistency Trägermaterials erreicht unter Umgehung der genannten Nachteile durch Misch- und Dosierfehler und Kontamination.

Mit den erfindungsgemäßen zweistufig härtbaren mischergängigen Materialien ist es möglich, knetbare Materialien aus automatischen Misch- und Dosiersystemen auszutragen. Die Viskositätserhöhung durch den zweistufigen Reaktionsmechanismus lässt sich so aussteuern, dass ein zunächst mischergängiges Mehrkomponenten Abformmaterial, das sich aus manuellen und automatischen Misch- und Dosiersystemen austragen lässt, während und nach der Mischzeit (nach ISO 4823) schnell in eine knetbare Konsistenz übergeht. Diese knetbare Konsistenz wird bis zum Ende der Gesamtverarbeitungszeit des Abformmaterials aufrecht erhalten.

Das erfindungsgemäße mischergängige Mehrkomponenten Abformmaterial kann so hergestellt werden, dass ein knetbares Abformmaterial nach Austragen aus einem manuellen oder automatischen Misch- oder Dosiersystem das klebfreie Verhalten eines klassischen Putty-Materials besitzt. Ein klebfreies Verarbeiten und Verstreichen des Abformmaterials mit dem Finger ist möglich. Bei der Sandwich-Abformtechnik kann der Anwender mit dem Finger die notwendige Mulde mit dem Finger eindrücken, ohne kleben zu bleiben.

Weiterhin ist es durch eine andere Ausführungsform der erfindungsgemäßen mischergängigen Abformmaterialeien möglich, besonders vorteilhafte Alginatersatzmaterialien für die Situationsabformung herzustellen. Hier wird die starke Konsistenzerniedrigung (Viskositätsanstieg) durch die erste Reaktionsstufe des zweistufigen Reaktionsmechanismus dazu genutzt, besonders standfeste und zähe Konsistenzen zu erhalten.

Diese alginatähnliche Konsistenz wird während der Gesamtverarbeitungszeit des Abformmaterials nach ISO 4823 aufrecht erhalten und ermöglicht dem Anwender vor dem Einbringen des Abformlöffels , das Material an den abzuformenden Zähnen mit dem Finger zu verstreichen, ohne dass das Material an den Fingern kleben bleibt.

Der beschriebene erfindungsgemäße Übergang der Mischungen von einem ersten Zustand zu einem zweiten Zustand höherer Viskosität zu einem dritten festen Zustand wird unter anderem durch folgende bevorzugte Ausführungsformen erreicht:

In einer erfindungsgemäßen Ausführungsform z.B. auf Polysiloxanbasis wird der Viskositätsanstieg der Abformmasse zur heavy bodied bzw. putty consistency erzielt, indem hauptsächlich α-Monoethinylpolysiloxane durch Hydrosilylierung an das Polymethylhydrogensiloxan angeknüpft werden. Aus der Polymethylhydrogensiloxankette entsteht durch das "Andocken" der Monoethinylpolysiloxane eine stark verzweigte Struktur, die man anschaulich als "Igelpolymer" beschreiben kann. Auf diese Weise werden Polymere mit sehr hoher Molmasse aufgebaut, die aber unter sich keine Vernetzung aufweisen.

Ein Zusatz von Dialkinylpolysiloxanen kann den Molmassensprung zusätzlich stark erhöhen.

Sobald der Großteil der zuerst reagierenden Alkinylpolydimethylsiloxane abreagiert hat, beginnt nach einer durch Inhibitoren einstellbaren Verarbeitungszeit der zweite Reaktionsschritt.

Hierbei reagieren die Dialkenylpolysiloxane mit den restlichen SiH-Gruppen des im Erstschritt hochverzweigten "Igelpolymers". Dieser zweite Schritt bei der dentalen Anwendung im Patientenmund führt zur Vernetzung und vollständigen Aushärtung der elastomeren Dentalabformmasse.

Durch gezielten Einsatz von Inhibitoren kann die jeweilige Kinetik im ersten und im zweiten Reaktionsschritt nach den jeweiligen Praxisanforderungen geregelt werden.

Eine weitere Ausführungsform der Erfindung verwendet für den ersten Reaktionsschritt Dialkinylpolysiloxane. Die Reaktion der Dialkinylpolysiloxane mit einem Überschuss an linearen Dihydrogenpolysiloxanen zu langen linearen Polymerketten von hoher Molmasse bewirkt den gewünschten Viskositätssprung, der gleichbedeutend mit dem Übergang vom ersten Zustand zum zweiten Zustand ist.

Sobald der Großteil der zuerst reagierenden Alkinylpolysiloxane abreagiert hat, beginnt der zweite Reaktionsschritt. Hierbei reagieren verzweigte Polysiloxane mit mindestens drei Vinylgruppen mit den SiH - Gruppen der Dihydrogenpolysiloxane, die nun am Polymerende sitzen.

Dieser zweite Schritt bei der dentalen Anwendung im Patientenmund führt zur Vernetzung und vollständigen Aushärtung der elastomeren Dentalabformmasse.

Ein Zusatz von Tri- oder Polyhydrogensiloxanen kann den Molmassensprung zusätzlich stark erhöhen.

In einer weiteren Ausführungsform der Erfindung reagieren in einem ersten Reaktionsschritt eine exakt definierte Konzentration eines Dialkinylpolysiloxans mit einem Polyhydrogensiloxan, bevorzugt mit hoher Molmasse (1000 bis 450 000 g/mol). Durch die Verknüpfung werden schnell Polysiloxane mit hoher Molmasse aufgebaut. Dies äußert sich im Verhalten der Abformmasse durch einen erfindungsgemäßen Viskositätssprung

Nachdem die gesamten Ethinylsiloxane abreagiert sind, reagieren in einem zweiten Reaktionsschritt die überschüssigen SiH - Gruppen mit den vorhandenen Dialkenylpolysiloxanen. Dieser zweite Schritt bei der dentalen Anwendung im Patientenmund führt zur Vernetzung und zur vollständigen Aushärtung der elastomeren dentalen Abformmasse.

Der erste Reaktionsschritt ist bei den drei zuletzt oben aufgeführten Ausführungsformen eine Hydrosilylierung zwischen der Alkinylgruppe und der SiH-Gruppe. In diesem Reaktionsschritt darf nur minimale Vernetzung auftreten, da sich sonst elastische Anteile schon während der Verarbeitungszeit aufbauen. Die entstandenen elastischen Anteile würden während der Verarbeitungszeit bei der Abdrucknahme zu Verpressungen (endogene Spannungen) führen, die in der Konsequenz zu nicht passendem Zahnersatz führen würden.

In einer weiteren Ausführungsform der Erfindung reagieren in einem ersten Reaktionsschritt ein α, ω - Dihydroxy- oder Dialkoxypolysiloxan mit einem Titanorthoester, wie beispielsweise Titan (IV) tetrapropylat. Durch die auftretende Kondensationsreaktion werden relativ schnell Polysiloxane mit hoher Molmasse aufgebaut. Dies äußert sich im Verhalten der Abformmasse durch einen Viskositätssprung.

Nachdem die Hydroxy- oder Alkoxysiloxane abreagiert sind, reagieren in einem zweiten Reaktionsschritt in einer platinkatalysierten Additionsreaktion Organohydrogensiloxane mit vorhandenden Dialkenylpolysiloxanen. Dieser zweite Schritt bei der dentalen Anwendung im Patientenmund führt zur Vernetzung und zur vollständigen Aushärtung der elastomeren dentalen Abformmasse. Der beschriebene Mechanismus kann natürlich auch in umgekehrter Weise ablaufen, d.h. zuerst die Addition und dann die Kondensation, abhängig von Art und Konzentration der Katalysatoren und Art und Konzentration der verwendeten Inhibitoren.

In einer weiteren Ausführungsform der Erfindung reagieren in einem ersten Reaktionsschritt sowohl die Alkinylgruppe eines Dialkinylpolysiloxans mit einem Polyhydrogensiloxan als auch ein α, ω - Dihydroxy- oder Dialkoxysiloxan mit einem Titanorthoester. Durch die auftretende Alkinadditionsreaktion und die Kondensationsreaktion werden schnell Polysiloxane mit hoher Molmasse aufgebaut. Dies äußert sich im rheologischen Verhalten der Abformmasse durch einen Viskositätssprung.

Nachdem die Alkinylgruppen und die Hydroxy- oder Alkoxygruppen abreagiert sind, reagieren in einem weiteren Reaktionschritt in einer platinkatalysierten Additionsreaktion Organohydrogenpolysiloxane mit vorhandenen Dialkenylpolysiloxanen. Dieser Schritt bei der dentalen Anwendung im Patientenmund führt zur Vernetzung und zur vollständigen Aushärtung der elastomeren, dentalen Abformmasse. Der beschriebene Mechanismus kann natürlich auch hinsichtlich der Kondensationsreaktion und der Additionsreaktion mit SiH und Alkenyl in umgekehrter Weise ablaufen, d.H. erst die Addition und dann die Kondensation, abhängig von Art und Konzentration der Katalysatoren und Art und Konzentration der Inhibitoren.

In einer weiteren erfindungsgemäßen Ausführungsform z.B. auf Polysiloxanbasis wird der Viskositätsanstieg der Abformmasse zur heavy bodied bzw. Putty consistency erzielt, indem hauptsächlich α-Monohydroxypolysiloxane durch Kondensation an das z.B. Titanalkoxid angeknüpft werden. So entsteht aus dem Titanalkoxid durch das "Andocken" der Monohydroxypolysiloxane eine verzweigte Struktur, die man anschaulich und bildlich als "Igelpolymer" bezeichnen kann. Auf diese Weise werden Polymere mit sehr hoher Molmasse aufgebaut, die aber unter sich keine Vernetzung aufweisen. Ein Zusatz von Dihydroxypolysiloxan kann den Molmassensprung zusätzlich stark erhöhen.

Sobald der Großteil der zuerst reagierenden Hydroxypolydimethylsiloxane abreagiert hat, beginnt nach einer durch Inhibitoren einstellbaren Verarbeitungszeit der zweite Reaktionsschritt.

Hierbei reagieren die Dialkenylpolysiloxane mit den vorhandenen SiH-Gruppen des Organohydrogenpolysiloxans. Dieser zweite Schritt bei der dentalen Anwendung im Patientenmund führt zur Vernetzung und vollständigen Aushärtung der elastomeren Dentalabformmasse.

Durch gezielten Einsatz von Inhibitoren kann die jeweilige Kinetik im ersten und im zweiten Reaktionsschritt nach den jeweiligen Praxisanforderungen geregelt werden.

Werden anstelle der Monohydroxysiloxane ω-Hydroxy-ω-vinylpolysiloxane eingesetzt, können die entstandenen Igelpolymere durch die noch frei verfügbaren Si-Vinylgruppen im zweiten Reaktionsschritt durch Hydrosilylierung in die Polymermatrix eingebaut werden.

### Beispiele

### Beispiel 1

### Kinetische Untersuchungen an Modellsystemen zur Reaktionsgeschwindigkeit von Ethinyl- , und Vinylgruppen bei der platinkatalysierten Hydrosilylierung (erfindungsgemäß).

Aufbau und Durchführung der kinetischen Versuche:GC-Analyse: 30 m FS-Kapilarsäule, Film 1,0 µm, belegt mit 100% Polydimethylsiloxan (Econo-Cap Kapillare EC-1 von Alltech GmbH). Säulenvordruck 0,35 bar H₂. Temperatur Splitinj. und FID bei 250°C.

Katalyseansatz: Vorlage Pt-Katalysator, Zugabe Pentamethyldisiloxan, Rühren bei 33°C für 20 bis 40 min. Zuspritzen des ungesättigten Substrats. Probenahme von 1ml-Proben zum jeweils zu untersuchenden Reaktionszeitpunkt, Verdünnen mit 4 ml einer TMEDA-Lösung (enthält 0,5 mmol TMEDA/4 ml) zum Einfrieren der Reaktion. Einspritzen von 1 µl dieser Lösung für die GC-Analyse.

### a) Kinetische Untersuchung der Hydrosilylierung von Vinyltrimethylsilan

0,5 g (5 mmol) Vinyltrimethylsilan werden nach der oben beschriebenen Methode mit 1,5 g Pentamethyldisiloxan (10 mmol) in 5,2 g Trimethylsilyl-endgestopptem Polydimethylsiloxan (MD₁₄M) als Lösungsmittel in Gegenwart von 0,1 mg Platin-Katalysator (in Form von Divinyltetramethyldisiloxan-Platin-Komplex, 1%ig in Xylol) umgesetzt, was einem Gehalt an reinem Platin von 50 ppm entspricht. Der Reaktionsverlauf läßt sich GC-analytisch nicht verfolgen, da das Vinyltrimethylsilan nach ca. 30 s unter Überhitzung vollständig umgesetzt ist.

### b) Kinetische Untersuchung der Hydrosilylierung von Ethinyltrimethylsilan

0,5 g (5 mmol) Ethinyltrimethylsilan werden nach der oben beschriebenen Methode mit 3,0 g (20mmol) Pentamethyldisiloxan in 5,2 g Trimethylsilyl-endgestopptem Polydimethylsiloxan (MD₁₄M) als Lösungsmittel in Gegenwart von 0,1 mg Platin-Katalysator (eingesetzt in Form von Divinyltetramethyldisiloxan-Platin-Komplex, 1%ig in Xylol) umgesetzt, was einem Gehalt an reinem Platin von 50 ppm entspricht. Das Reaktionsgemisch wird zu verschiedenen Zeitpunkten über GC-Analyse untersucht. Es wird ein Mono- und ein Diadditionsprodukt gebildet. Nach 1000 s ist Ethinyltrimethylsilan vollständig verbraucht unter Bildung von 80% Monoadditionsprodukt und 20% Diadditionsprodukt. Nach 3 Tagen bei 35°C ist das Diadditionsprodukt zu 98% gebildet worden.

### c) Kinetische Untersuchung der Hydrosilylierung eines Gemisches aus Ethinyltrimethylsilan und Vinyltrimethylsilan

Ein Gemisch aus 0,3 g Vinyltrimethylsilan und 0,3 g Ethinyltrimethylsilan wird nach der oben beschriebenen Methode mit 1,5 g Pentamethyldisiloxan in 5,2 g Trimethylsilyl-endgestopptem Polydimethylsiloxan (MD₁₄M) als Lösungsmittel in Gegenwart von 0,1 mg Platin-Katalysator (eingesetzt in Form von Divinyltetramethyldisiloxan-Platin-Komplex, 1%ig in Xylol) umgesetzt, was einem Gehalt an reinem Platin von 50 ppm entspricht. Ethinyltrimethylsilan wird bei dieser Reaktion schneller verbraucht als Vinyltrimethylsilan. Nach 600 s ist das gesamte Ethinyltrimethylsilan zu 80% Monoadditionsprodukt und 20% Diadditionsprodukt umgewandelt. Die Teilreaktion zwischen Vinyltrimethylsilan und Pentamethyldisiloxan ist dagegen gegenüber Beispiel 1a) deutlich verlangsamt. Nach 1500 s ist das gesamte Vinyltrimethylsilan verbraucht.

### Diskussion der Ergebnisse der kinetischen Untersuchungen aus den Beispielen 1a) bis c):

Die Hydrosilylierung von Ethinyltrimethylsilan ist im Einzelexperiment langsamer als die Hydrosilylierung von Vinyltrimethylsilan (Beispiele 1a und 1b).

Wird eine Abmischung aus Ethinyltrimethylsilan und Vinyltrimethylsilan bei der Hydrosilylierung eingesetzt (Beispiel 1c), so reagiert zunächst Ethinyltrimethylsilan bevorzugt ab. Dies ist darauf zurückzuführen, dass Ethinyltrimethylsilan fester oder schneller am Platin koordiniert als Vinyltrimethylsilan. Erst wenn Ethinyltrimethylsilan fast vollständig verbraucht ist, tritt eine merkliche Reaktion des Vinyltrimethylsilans ein.

Dieser zweistufige Reaktionsverlauf kann erfindungsgemäß zur Herstellung zweistufig härtbarer Siliconmaterialien genutzt werden, indem die Hydrosilylierung von Ethinylgruppen zum Aufbau hoher Molekulargewichte bzw. Viskositäten genutzt wird und die zeitlich versetzte Hydrosilylierung von Vinylgruppen zur Vernetzung genutzt wird.

### Beispiel 2

### Verhalten von Ethinyltrimethylsilan als Modellverbindung in einem additionsvernetzendem Siliconmaterial (erfindungsgemäß).

a) 2 Teile Ethinyltrimethylsilan werden in einem Mischer mit 38 Teilen eines vinylendgestoppten Polydimethylsiloxans mit einer Viskosität von 1000 mPas und einem Vinylgehalt von 0,13 mmol/g gelöst. Man erhält eine farblose klare 5%ige Lösung.
b) In 100 Teile der Komponente 2 (SiH-Komponente) einer nicht inhibierten mittelfließenden Abformmasse werden 3 Teile der unter a) hergestellten Mischung in einem Mischer homogen eingearbeitet.
c) 50 Teile der unter b) hergestellten Komponente 2 werden mit 50 Teilen der Komponente 1 (Pt-Komponente) einer mittelfließenden Abformmasse aus einer Zweikammerkartusche über einen statischen Mischer homogen gemischt. Man erhält eine mittelfließende Abformmasse (nach DIN EN 24823) mit einer Gesamtverarbeitungszeit von einer Minute und einem Abbindeende von 2,5 Minuten.

Beispiel 2 zeigt auf, dass Si-Ethinylgruppen in einem additionsvernetzenden Siliconmaterials keinen stark inhibitierenden Einfluss auf das Abbindeverhalten hat. Die Konzentration an Ethinyl wurde mit 1,5 mmol/100 g so gewählt, wie sie bei Verwendung von Monoethinylpolydimethylsiloxan zur Einstellung der gewünschten Molmassenerhöhung notwendig wäre. Der Übergang von der medium bodied zur putty consistency ist nach dieser Modellreaktion nach einer Minute abgeschlossen.

### Beispiel 3

### Modellversuche zu zweistufig härtbaren additions- und kondensationsvernetzenden mischergängigen Siliconmaterialien (erfindungsgemäß)

a) In einem Vakuummischer werden 196 Teile eines α, ω - Divinylpolydimethylsiloxans mit einer Viskosität von 1000 mPas (bei 23°C), einer Molmasse von ca. 15000 g/mol und einem Vinylgehalt von 0,13 mmol/g mit 2,5 Teilen eines in Divinylpolydimethylsiloxan gelösten Platin-SiloxanKomplexes mit einem Gehalt an reinem Platin von 1% und 1,5 Teilen Titan (IV) tetrapropylat homogen gemischt. Man erhält eine sehr dünnfließende Abmischung mit einer Viskosität von ca. 800 mPas (bei 23°C), die die Komponente 1 des erfindungsgemäß in zwei Stufen härtbaren additions- und kondensationsvernetzenden Siliconmaterials darstellt.
b) In einem Vakuummischer werden 113 Teile eines Polymethylhydrogensiloxans mit einer Viskosität von 200 mPas (bei 23°C), einer Molmasse von ca. 7000 g/mol und einem SiH-Gehalt von 1,8 mmol/g mit 83 Teilen eines α, ω -Dihydroxypolydimethylsiloxans mit einer Viskosität von 2000 mPas (bei 23°C), 0,34 Teilen 1,2 Divinyltetramethyldisiloxan und 3 Teilen α, ω- Divinylpolydimethylsiloxan mit einer Viskosität von 1000 mPas (bei 23°C) homogen gemischt. Man erhält eine sehr dünnfließende Abmischung mit einer Viskosität von 500 mPas (bei 23°C), die die Komponente 2 des erfindungsgemäß in zwei Stufen härtbaren additions- und kondensationsvernetzenden Siliconmaterials darstellt.
c) 3 Teile der Komponente 1 aus Beispiel 3 a) und 1 Teil der Komponente 2 aus Beispiel 3b) werden auf einem Mischblock mit Hilfe eines Mischspatels innerhalb 30 s gemischt. Das Material erfährt innerhalb kurzer Zeit eine Viskositätserhöhung. Die Viskosität der Mischung beträgt unmittelbar nach Mischen 20 000 mPas. Diese hohe Viskosität wird über einen Zeitraum von ca. fünf Minuten gehalten. Gegen Ende dieser Verarbeitungszeit setzt die zweite Reaktionsstufe ein und das Material härtet innerhalb 12 Minuten bei Raumtemperatur aus.

Dieses Beispiel verdeutlicht, dass es möglich ist mit der erfindungsgemäßen Zusammensetzung einen zweistufigen Reaktionsmechanismus auszubilden, der ein anfänglich sehr dünnfließendes mischergängiges Material durch einen Viskositätssprung in ein zähfließenderes Material übergehen lässt, das nach einer angemessenen Verarbeitungszeit in einer zweiten Reaktionsstufe zu einem elastomeren Festkörper vernetzt.

Dieses Verhalten kann erfindungsgemäß zur Herstellung von zähfließenden bis knetbaren Abformmaterialien ausgenutzt werden, die sich leicht aus marktgängigen Misch- und Dosiergeräten austragen lassen. Überraschenderweise zeigt sich beim erfindungsgemäßen Beispiel 3c, dass für die Reaktion des Kondensationsvernetzers mit den Polymeren mit SiOH-Gruppen kein Wasserzusatz erforderlich ist. Im Gegenteil verhindert auch ein geringer Zusatz von Wasser das Auftreten eines Viskositätsprungs. Im Gegensatz dazu ist bei den literaturbekannten Systemen für (Zahn)abformmaterialien ein Zusatz von Wasser erforderlich ("Silicone Chemie und Technologie", Vulkan Verlag Essen 1989, S. 55; W. Noll, "Chemie und Technologie der Silicone", 2. Auflage, Verlag Chemie 1968, Seite 340).

Die Herstellung entsprechender zweistufig härtbarer Abformmaterialien wird in den Beispielen 4 und 5 beschrieben.

### Beispiel 4

Formulierungen von zweistufig härtbaren additionsvernetzenden und kondensationsvernetzenden mischergängigen Siliconmaterialien (erfindungsgemäß).
a) 67 Teile Quarzmehl, 1 Teil hochdisperse Kieselsäure mit einer BET-Oberfläche von 200 m²/g und 2 Teile eines Molekularsiebs, werden in einem Kneter mit 8 Teilen Mineralöl mit 200 mPas, 0,5 Teilen eines in Divinylpolysiloxan gelösten Pt-Siloxankomplexes mit einem Gehalt an reinem Platin von 1,0 % , 21,0 Teilen eines Vinyl-endgestoppten Polydimethylsiloxans mit einer Viskosität von 1000 mPas (bei 23°C) und 0,5 Teilen Titan(IV)tetrapropylat homogen gemischt.
b) 67 Teile Quarzmehl, 1 Teil hochdisperse, hydrophobierte Kieselsäure mit einer BET - Oberfläche von 200 m²/g und 2 Teile eines Molekularsiebs werden in einem Kneter mit 8 Teilen Mineralöl mit einer Viskosität von 200 mPas (bei 23°C), 5 Teilen eines Polymethylhydrogensiloxans mit einer Molmasse von ca. 7000 g/mol und einem SiH-Gehalt von 1,8 mmol/g und 7 Teile eines Divinylpolydimethylsiloxan mit einer Viskosität von 1000 mPas (23°) und einem Vinylgehalt von 0,13 mmol/g, 10 Teilen α, ω- Dihydroxypolydimethylsiloxan und mit einer Viskosität von 2000 mPas und einem SiOH-Gehalt von 0,1 mmol/g homogen gemischt. Man erhält eine mittelfließende Paste, die die Komponente 2 (SiH-Komponente) des erfindungsgemäßen in zwei Stufen härtbaren Siliconmaterials darstellt.
c) 50 Teile der Komponente 1 aus Beispiel 4a) und 50 Teile der Komponente 2 aus Beispiel 4b) werden aus einer Zweikammerkartusche mit einer Dosierpistole über einen statischen Mischer homogen gemischt. Da das Abformmaterial in seinen Einzelkomponenten in mittelfließender Konsistenz vorliegt, lässt es sich leicht über bekannte elektrisch-, pneumatisch- oder handbetriebene Misch- und Dosiersysteme (Fa. Mixpac, EP 0615787 A1) austragen. Während und nach dem Ende des Mischens und setzt die erste Reaktionsstufe ein. Die Mischung wird nach dem Mischen ausgetragen, so dass die erste Reaktionsstufe weitgehend außerhalb des Mischers stattfindet.

Das Material erfährt innerhalb kurzer Zeit eine Viskositätserhöhung, und geht in ein putty consistency nach ISO 4823 Vorabformmaterial über. Diese putty consistency wird über einen gewissen Zeitraum gehalten. Gegen Ende der Verarbeitungszeit wird das Abformmaterial in den Mund eingebracht. Nun setzt die zweite Reaktionsstufe ein und das Material härtet vollständig aus. Das erhaltene Vulkanisat weist die mechanischen Eigenschaften eines Putty-Materials nach ISO 4823 auf.

### Beispiel 5

### Formulierungen von zweistufig härtbaren additionsvernetzenden und kondensationsvernetzenden mischergängigen Siliconmaterialien (erfindungsgemäß).

a) 67 Teile Quarzmehl, 1 Teil hochdisperse hydrophobierte Kieselsäure mit einer BET - Oberfläche von 200 m²/g und 2 Teile eines Molekularsiebs werden in einem Kneter mit 8 Teilen Mineralöl mit 200 mPas, 6 Teilen α, ω-Diethinylpolydimethylsiloxan mit einer Viskosität von 1000 mPas, einer Molmasse von ca. 15000 g/mol und einem Ethinylgehalt von ca. 0,13 mmol/g und 15 Teilen eines α, ω-Divinylpolydimethylsiloxans, das eine Viskosität von 1000 mPas, eine Molmasse von ca. 15000 g/mol und einem Vinylgehalt von 0,13 mmol/g aufweist, 0,5 Teilen eines in Vinylsiloxan gelösten Platin - Siloxan - Komplexes mit einem Gehalt an reinem Platin von 1 % und 0,5 Teilen Titan(IV) tetrapropylat homogen gemischt. Man erhält eine mittelfließende Paste, die die Komponente 1 des erfindungsgemäß in zwei Stufen härtbaren additionsvernetzenden Siliconmaterials darstellt.
b) 67 Teile Quarzmehl, 1 Teil hochdisperse, hydrophobierte Kieselsäure mit einer BET-Oberfläche von 200 m²/g und 2 Teile eines Molekularsiebes werden in einem Kneter mit 8 Teilen Mineralöl mit 200 mPas, 6 Teilen Polymethylhydrogensiloxan mit einer Molmasse von 7000 g/mol und einem SiH-Gehalt von 1,8 mmol/g und 6 Teilen eines α, ω-Divinylpolydimethylsiloxans, das eine Viskosität von 1000 mPas, eine Molmasse von ca. 15000 g/mol und einen Vinylgehalt von 0,13 mmol/g aufweist, und 10 Teilen eines α, ω-Dihydroxypolydimethylsiloxans mit einer Viskosität von 2000 mPas (bei 23°C) homogen gemischt. Man erhält eine mittelfließende Paste, die die Komponente 2 des erfindungsgemäß in zwei Stufen härtbaren additionsvernetzenden und kondensationsvernetzenden Siliconmaterials darstellt.
c) 50 Teile der Komponente 1 aus Beispiel 5a) und 50 Teile der Komponente 2 aus Beispiel 5b) werden aus einer Zweikammerkartusche mit einer Dosierpistole über einen statischen Mischer homogen gemischt. Da das Abformmaterial in seinen Einzelkomponenten in mittelfließender Konsistenz vorliegt, lässt, es sich leicht über bekannte elektrisch-, pneumatisch- oder handbetriebene Misch- und Dosiersysteme (Fa. Mixpac, EP 0615787 A1) austragen. Nach dem Mischen setzt die erste Reaktionsstufe ein. Die Mischung wird nach dem Mischen ausgetragen, so dass die erste Reaktionsstufe weitgehend außerhalb des Mischers stattfindet. Das Material erfährt innerhalb kurzer Zeit eine Viskositätserhöhung, und geht in eine putty consistency nach ISO 4823 über. Diese putty consistency wird über einen gewissen Zeitraum gehalten. Gegen Ende der Verarbeitungszeit wird das Abformmaterial in den Mund eingebracht. Nun setzt die zweite Reaktionsstufe ein und das Material härtet vollständig aus. Das erhaltene Vulkanisat weist die mechanischen Eigenschaften eines Putty-Materials nach ISO 4823 auf.

Die Ergebnisse werden im Zusammenhang mit Beispiel 6 diskutiert.

### Beispiel 6

Formulierungen von zweistufig härtbaren additions- und kondensationsvernetzenden mischergängigen Siliconmaterialien (erfindungsgemäß).
a) 47 Teile Quarzmehl und 15 Teile hochdisperse hydrophobierte Kieselsäure mit einer BET - Oberfläche von 200 m²/g werden in einem Kneter mit 18 Teilen Mineralöl mit 200 mPas, und 15 Teilen Vaseline, 3 Teilen eines in Vinylsiloxan gelösten Platin - Siloxan - Komplexes mit einem Gehalt an reinem .Platin von 1 % und 2 Teilen Titan (IV) tetrabutylat homogen bemischt. Man erhält eine mittelfließende Paste, die die Komponente 1 des erfindungsgemäß in zwei Stufen härtbaren additionsvernetzenden Siliconmaterials darstellt.
b) 66 Teile Quarzmehl, 2,5 Teil hochdisperse, hydrophobierte Kieselsäure mit einer BET - Oberfläche von 200 m²/g werden in einem Kneter mit 7 Teilen Polymethylhydrogensiloxan mit einer Molmasse von 7000 g/mol und einem SiH-Gehalt von 1,8 mmol/g und 17 Teilen eines α, ω-Divinylpolydimethylsiloxan-Gemisches, das eine Viskosität von 1200 mPas und einen Vinylgehalt von 0,13 mmol/g aufweist, 4 Teile α-Hydroxy-ωvinylpolydimethylsiloxan mit einer Viskosität von 5000 mPas und 2 Teilen eines α, ω-Dihydroxypolydimethyl-siloxans mit einer Viskosität von 5000 mPas (bei 23°C) homogen gemischt. Man erhält eine mittelfließende Paste, die die Komponente 2 des erfindungsgemäß in zwei Stufen härtbaren additionsvernetzenden und kondensationsvernetzenden Silicon materials darstellt.
c) 1 Teil der Komponente 1 aus Beispiel 6a) und 5 Teile der Komponente 2 aus Beispiel 6b) werden aus einer Zweikammerkartusche mit einer Dosierpistole über einen statischen Mischer homogen gemischt. Da das Abformmaterial in seinen Einzelkomponenten in mittelfließender Konsistenz vorliegt, lässt es sich leicht aus Kartuschen und Schlauchbeuteln über bekannte elektrisch-, (Fa. Espe, Pentamix, EP 0492413 D1; Fa. DMG, Mixstar, DE 10013812 A1, Fa. Mixpac EP 0956908 A1) pneumatisch- oder handbetriebene Misch- und Dosiersysteme (Fa. Mixpac, EP 0615787 A1) austragen. Während und nach dem Mischen setzt die erste Reaktionsstufe ein.

Die Mischung wird nach dem Mischen ausgetragen, so dass die erste Reaktionsstufe weitgehend außerhalb des Mischers stattfindet. Das Material erfährt innerhalb kurzer Zeit eine Viskositätserhöhung, und geht in eine putty consistency nach ISO 4823 über. Diese putty consistency wird über einen gewissen Zeitraum gehalten. Gegen Ende der Verarbeitungszeit wird das Abformmaterial in den Mund eingebracht. Nun setzt die zweite Reaktionsstufe ein und das Material härtet vollständig aus. Das erhaltene Vulkanisat weist die mechanischen Eigenschaften eines Putty-Materials nach ISO 4823 auf:

| | |
|---|---|
| Verformung unter Druck: | 2,5% |
| Rückstellung nach der Verformung: | 99,4% |
| Lineare Maßänderung: | -0,2% |
| Shore A-Härte (DIN 53505) | 70 |
| Reißfestigkeit (DIN 53504) | 160 N/cm² |

### Diskussion der Beispiele 5 und 6:

Bei der erfindungsgemäßen Kombination der Additionsvernetzung von Alkenylmit SiH- und der Kondensationsreaktion von SiOR mit Metallalkoxiden mit oder ohne der Addition von Alkinyl- mit SiH zeigt sich, wie in den Beispielen 5c und 6c beschrieben, dass die Additionsreaktion und die Kondensationsreaktion praktisch unabhängig voneinander ablaufen und keinen wechselseitigen negativen Einfluss aufeinander haben. Die für einen Fachmann zu erwartende und literaturbekannte (Silicone Chemie und Technologie", Vulkan Verlag Essen 1989, S.55; W. Noll, "Chemie und Technologie der Silicone", 2. Auflage, Verlag Chemie 1968, Seite 340) Reaktion zwischen SiOH und SiH in Gegenwart von Platin unter Wasserstoffentwicklung (Gasblasenbildung) tritt hier überraschenderweise nicht auf.

Die o.g. erfindungsgemäße Kombination zeigt eine lineare Maßänderung nach ISO 4823 in der selben Größenordnung wie bei den marktüblichen additionsvernetzenden Siliconen. Die lineare Maßänderung liegt zwischen -0,1% und -0,3%, während die marktüblichen kondensationsvernetzenden Silicone zwischen -0,6% und -1,5% liegen (J. Wirz, "Abformung in der zahnärztlichen Praxis", Gustav Fischer Verlag 1993, S. 56, 57; Dr. Wöstmann "Zum derzeitigen Stand der Abformung in der Zahnheilkunde", Habilitationsschrift, Universität Münster, S.18ff). Das erfindungsgemäße Beispiel 6c weist eine lineare Maßänderung von -0,2% auf.

Die Einzelkomponenten der Beispiele 5a und 6a, in denen eine Vernetzer/Katalysator-Kombination, bestehend aus Metallalkoxiden, insbesondere Titanate und Hydrosilylierungskatalysatoren, insbesondere Pt-Komplexe vorliegt, zeigen eine hohe Lagerstabilität in geeigneten Primärverpackungen sowohl bei Raumtemperatur als auch bei Thermostresstestbedingungen bei 60 °C. Auf diese Weise ist die in der Praxis geforderte Haltbarkeit des Verkaufsproduktes von mindestens 6 Monaten gegeben.

Der in den Einzelkomponenten der Beispiele 5a und 6a enthaltene Kondensationsvernetzer (Metallalkoxide, insbesondere Titanate) weist in der erfindungsgemäßen Zusammensetzung eine hohe, für die Praxisbedingungen ausreichende Lagerstabilität in der geeigneten Primärverpackung, wie z.B. Aluminium-beschichteten Folienschlauchbeuteln oder wasserdampfundurchlässigen Kunststoffkartuschen, auf. Das heißt, die Aktivität der kondensationsvernetzenden Metallalkoxide, insbesondere Titanate, führt trotz des Vorhandenseins potentieller Wasserspuren zu einem ausreichenden Molmassenanstieg und damit verbundenen Viskositätssprung. Auf diese Weise ist die in der Praxis geforderte Haltbarkeit des Verkaufsproduktes von mindestens 6 Monaten gegeben.

Überraschenderweise zeigen die Einzelkomponenten der Beispiele 5b und 6b, in denen Polymere mit SiH und SiOH-Gruppen nebeneinander vorliegen, keine Reaktion zur Knüpfung einer Si-O-Si-Bindung unter Wasserstoffabspaltung sowohl bei Raumtemperatur als auch unter Thermostresstestbedingungen bei 60°C, d.h. es konnte kein Viskositätsanstieg der o.g. Einzelkomponenten und kein Aufblähen der Primärverpackung durch Wasserstoffgase beobachtet werden.

### Beispiele 7

### Vergleichsbeispiel: handelsübliches knetbares Vorabformmaterial (nicht erfindungsgemäß).

Es werden handelsübliche Putty - Materialien Panasil Putty (Kettenbach # 7399) und Provil novo putty fast set (Heraeus - Kulzer, Lot.-Nr. 090554/090439) jeweils getrennt in Komponente 1 und Komponente 2 in Zweikammerkartuschen EP 0723807A2) bzw. Folienschlauchbeutel (vgl. EP-A-0541972, PCT/EP 98/0193) abgefüllt.

Ein Austragen der o.g. Puttymaterialien aus der Zweikammerkartusche mit einer Dosierpistole (Fa. Mixpac) über einen statischen Mischer (Fa. Mixpac MA 7,5 - 12-D) und ein Befüllen eines Konfektionsabformlöffels ist aufgrund der hohen Austragskräfte innerhalb der Gesamtverarbeitungszeit nach ISO 4823 nicht möglich.

Ein Austragen der o.g. Puttymaterialien aus einem Folienschlauch mit einem elektrischen betriebenen Misch- und Dosiergerät (Fa. Mixpac Typ BD 400-230-0000, Serial Nr. Vorserie 00029, EP 0956908 A1; Fa. Espe, Pentamix 2; EP-A-0492413) über einen dynamischen Mischer (EP-A-0492412) und ein Befüllen eines Konfektionsabformlöffels ist aufgrund der hohen Austrags- und Reibungskräfte innerhalb der Gesamtverarbeitungszeit nicht möglich.

Dieses Beispiel verdeutlicht, dass sich ein handelsübliches Putty-Material im Gegensatz zum erfindungsgemäßen Abformmaterial nicht mit automatischen Misch- und Dosiersystemen verarbeiten lässt.

### Beispiel 8

### Vergleichsbeispiel: handelsübliches Putty-Cartridge-Material (nicht erfindungsgemäß)

Untersucht wird das Handelsprodukt Reprosil Quixx Putty, Fa. Dentsply Caulk, in dessen Packungsbeilage folgende Aussage gemacht wird:

"Nach Austragen aus einer Doppelkammerkartusche mittels eines statischen Mischers geht es von einer sofortigen Mischungsviskosität über eine Tray-Viskosität in eine Putty-Viskosität eine Minute nach Mischbeginn über". Die ideale Putty-Viskosität wird zwischen 1 Minute 15 Sekunden und 1 Minute 30 Sekunden erreicht. Während dieses Zeitfensters verhält sich das Material wie ein traditionelles Putty-Material.

Bei dem o.g. Handelsprodukt handelt es sich um ein nach dem Stand der Technik bekanntes additionsvernetzendes Silicon, d.h. Siloxanpolymere mit Vinylgruppen reagieren mit Hydrogenpolysiloxanen und führen zu einer Quervernetzung.

Dies bedeutet, dass durch die laufende Vernetzung die Viskosität kontinuierlich ansteigt, ohne dass eine Putty ähnliche Konsistenz aufrechterhalten wird (im Gegensatz zum erfindungsgemäßen 2 - Stufen Reaktionsmechanismus).

Daraus ergeben sich folgende Konsequenzen:
Das Zeitfenster, in dem die Putty - Konsistenz aufrechterhalten wird, ist äußerst kurz und damit für den Anwender (Zahnarzt) nur schwer kalkulierbar (z.B. Einfluss der Aussentemperatur auf Kinetik).
Das kurze Zeitfenster von 15 Sekunden beinhaltet folgende Risiken:
Bei Unterschreitung der Zeitvorgabe von 1 Minute und 15 Sekunden, z.B. nach einer Minute ist die Mischungskonsistenz noch nicht ausreichend hoch, so dass bei Abdrucknahme nur ein geringer Druckaufbau durch das Abformmaterial erreicht wird.
Bei Überschreiten der Zeitvorgabe von 1 Minute und 30 Sekunden ist die Vernetzung schon sehr weit fortgeschritten, so dass die elastischen Eigenschaften gegenüber den plastischen Eigenschaften überwiegen und somit bei Abdrucknahme das Risiko endogener Spannungen und Verpressungen extrem hoch ist.
Dies führt zwangsläufig zu nicht akzeptablen Abformergebnissen.

Darüber hinaus zeigt das Handelsprodukt Reprosil Quixx Putty in dem ausgewiesenen Zeitfenster zwischen einer Minute und 15 Sekunden und einer Minute und 30 Sekunden beim Handling zwischen den Fingern nicht die vom traditionellen Putty gewohnten knetbaren und nicht klebrigen Eigenschaften, sondern verhält sich wie ein mittelfließendes Abformmaterial, das nach dem einstufigen Hydrosilylierungsmechanismus aushärtet und sich in der Übergangsphase vom plastischen zum elastischen Zustand befindet. Es fühlt sich zwischen den Fingern klebrig an.

Die Beispiele 3 bis 5 zeigen auf, dass es mit den erfindungsgemäß zusammengesetzten Siliconmaterialien möglich ist, ein Material zu schaffen, das in seinen Einzelkomponenten in dünn- bis zähfließender Konsistenz vorliegt, sich leicht über bekannte elektrisch-, pneumatisch- oder handbetriebene Misch- und Dosiersysteme austragen lässt und innerhalb kurzer Zeit eine Viskositätserhöhung erfährt, (d.h. die Konsistenz der Abformmasse während und nach dem Mischen gemäß ISO 4823 geht von light bodied consistency (Typ 3) bis medium bodied consistency (Typ 2) in eine heavy bodied consistency (Typ 1) bzw. putty consistency (Typ 0) oder von einer heavy bodied consistency (Typ 1) in eine putty consistency (Typ 0) über).

Das erfindungsgemäße Siliconmaterial vereinigt die Vorteile der Putty Cartridge Materialien, wie automatische Misch- und Dosierbarkeit mit den Vorteilen der klassischen knetbaren Trägermaterialien wie Stempeldruckaufbau beim Abformen, Klebfreiheit im puttyartigen Zustand und angemessene Gesamtverarbeitungszeit durch einen zweistufigen Reaktionsmechanismus und vermeidet dadurch die oben beschriebenen Nachteile der Trägermaterialien nach dem Stand der Technik.

### Beispiel 9

Um den Begriff "mischergängig" genauer zu definieren, wurde die Austragbarkeit von Materialien nach dem Stand der Technik bestimmt. Die Austragbarkeit wurde für 7 Materialien jeweils mit einem automatischen und einem manuellen Misch- und Dosiersystem bestimmt. Die Ergebnisse werden in Tabelle 1 zusammengefaßt.

**Tabelle 1: Mischergängigkeit von Materialien nach dem Stand der Technik**

| Materialtyp nach ISO 4823 | Konsistenz der Mischung nach ISO 4823 | Austragbarkeit aus automatischen Mischund Dosiersystemen ¹⁾ | Austragbarkeit aus manuellen Mischund Dosiersystemen ²⁾ |
|---|---|---|---|
| putty ³⁾ | 22 mm | nicht austragbar | nicht austragbar |
| putty ⁴⁾ | 24 mm | nicht austragbar | nicht austragbar |
| putty ¹⁰⁾ | 27 mm | extrem schwer austragbar | nicht austragbar |
| heavy bodied ⁵⁾ | 26 mm | sehr schwer austragbar | nicht austragbar |
| heavy bodied ⁶⁾ | 28 mm | schwer austragbar | nicht austragbar |
| heavy bodied ⁷⁾ | 30 mm | akzeptabel | sehr schwer austragbar |
| heavy bodied ⁸⁾ | 32 mm | akzeptabel | schwer austragbar |
| heavy bodied ⁹⁾ | 34 mm | akzeptabel | akzeptabel |

| | | | |
|---|---|---|---|
| 1) Pentamix 1, Fa. Espe, dynamischer Mischer, Kartusche mit Folienschlauch Pentamix II, Fa. Espe, dynamischer Mischer, Kartusche mit Folienschlauch Mixstar, Fa. DMG, dynamischer Mischer, Kartusche mit Folienschlauch BDG 400 Bulk Dispenser (Prototyp), Fa. Mixpac, dynamischer Mischer, Kartusche CDB 400-01-V02 2) System S 50, Fa. Mixpac, statischer Mischer MB 7,5-11-D, Kartusche CS 050-01-09, Dispenser DS 50-01-00 System 50, Fa. Mixpac, statischer Mischer MA 7,5-12-D, Kartusche CD 051-01-09, Austraggerät DM 51-KE-1 Pa. TAH, statischer Mischer 190-712 D, Kartusche BS-101-01, Austragpistole DK-104 3) Panasil Putty regular set, Kettenbach 4) Panasil Putty soft, Kettenbach 5) Versuchsprodukt Panasil Plus heavy, Kettenbach 6) Versuchsprodukt Panasil Plus heavy, Kettenbach 7) Versuchsprodukt Panasil Plus heavy, Kettenbach 8) Panasil Tray fast set, Kettenbach 9) Kettosil, Kettenbach 10) Aquasil soft putty, Fa. DeTrey Dentsply, Pentamix II und BDG 400. (Beim Austragen wurde eine nicht akzeptable Temperaturerhöhung festgestellt. Die ausgetragene Volumenmenge betrug lediglich 17 ml nach 1 min und war damit viel zu niedrig). | | | |

### Beispiel 10

In Tabelle 2 wird die Austragsmenge verschiedener Handelsprodukte im Vergleich zur erfindungsgemäßen Abformmasse z.B. gemäß Beispiel 6c) mit unterschiedlichen Austraggeräten dargestellt.

Weiterhin werden die Mischungskonsistenzen und die Eigenschaften der "Knetbarkeit" und "Klebrigkeit" bewertet.

Als Fazit der Tabelle 2 wird deutlich, dass nur das erfindungsgemäße Beispiel 6c) eine extrem niedrige Mischungskonsistenz von 22 mm hat, knetbar und nicht klebrig ist, aber trotzdem in praxisgerechter Austragsmenge aus handelsüblichen Austragsgeräten auszutragen ist. Die Austragung des Materials mit einer Konsistenz von nur 22 mm ist erfindungsgemäß möglich, weil diese Konsistenz im Mischer erst durch kontinuierliche Zunahme der Viskosität erreicht wird. Daher hat im Mischer lediglich der Teil des Materials, der kurz vor der Austragung steht, eine solche relativ hohe Viskosität. Die relativ hohe Viskosität beeinträchtigt dann nicht mehr die Austragbarkeit. Dagegen ist ein Material nach dem Stand der Technik, das bereits mit einer Konsistenz von 22 mm in einen Mischer eingebracht wird, wie in Beispiel 9 gezeigt nicht austragbar.

**Tabelle 2**

| **Firma: Produkt und Charge:** | **Austragsmenge in g/min Austragsgerät:** | | | **konsistenz¹⁾ der Mischung** | **knetbar²⁾** | **klebrig³⁾** |
|---|---|---|---|---|---|---|
| | **Mixstar^{a)}** | **Penta I^{b)}** | **Penta II^{c)}** | **in mm** | | |
| Espe: Permadyne Penta H Kat 551, Base 270, Pentamatic | 48,6, | 57 | 82,4 | 32 | nein | ja |
| Espe: Impregum Penta Kat N80, Base E30, Pentamatic | 47,8 | 56,4 | 82,6 | 35 | nein | ja |
| Espe: Dimension Penta H Kat 459, Base 094 | 69,6 | 81 | 121,6 | 28 | nein | ja |
| Dentsply: Aquasil heavy body Kat 000327, Base 0003000367 | 57,6 | 68 | 93,6 | 29 | nein | ja |
| Coltene: President heavy body Kat 07, Base 11 | 58,2 | 69,4 | 92,2 | 29 | nein | ja |
| Kettenbach: Panasil Tray fast Kat 00021, Base 00021 | 63 | 75,6 | 118,6 | 32 | nein | ja |
| erfindungsgemäßes Beispiel 6c) | 54 | 67 | 103 | 22 | ja | nein |

| | | | | | | |
|---|---|---|---|---|---|---|
| a): Serien-Nr.:99-678, b): Serien-Nr.:535617, Espe; c): Serien-Nr.: 5106647, Espe 1): Bestimmung nach ISO 4823; 2): das Material ist sofort nach dem maschinellen Austragen zwischen den Fingern knetbar; 3): d.h.: Es ist nicht möglich, das Material sofort nach dem Anmischen zu kneten, aufgrund der Klebrigkeit. | | | | | | |

## Patentansprüche

1. Mehrkomponentensystem zur Abdrucknahme, enthaltend
(a) mindestens eine Verbindung mit mindestens zwei Alkenylgruppen,
(b) mindestens ein Organohydrogenpolysiloxan und
(c) mindestens einen Hydrosilylierungskatalysator,
**dadurch gekennzeichnet, dass** es
(d1) mindestens eine polymere Verbindung mit mindestens einer Alkinylgruppe und/oder
(d2) mindestens eine Verbindung mit mindestens einer Si - OR - Struktureinheit, wobei R = H, Alkyl, Alkoxyalkyl oder Acyl ist, und
wenn eine Verbindung (d2) mit mindestens einer Si - OR - Struktureinheit enthalten ist,
(e) mindestens ein Kondensationskatalysator und/oder -vernetzer enthält.

2. Mehrkomponentensystem zur Abdrucknahme nach Anspruch 1 mit mindestens zwei Komponenten A und B, **dadurch gekennzeichnet, dass** die Komponente A
(a) mindestens eine Verbindung mit mindestens zwei Alkenylgruppen und
(b) mindestens ein Organohydrogenpolysiloxan enthält,
sowie
(d1) mindestens eine polymere Verbindung mit mindestens einer Alkinylgruppe und/oder
(d2) mindestens eine Verbindung mit mindestens einer Si - OR - Struktureinheit, wobei R = H, Alkyl, Alkoxyalkyl oder Acyl ist, enthält,
die Komponente B
(c) mindestens einen Hydrosilylierungskatalysator und die Komponente A und/oder B, wenn eine Verbindung (d2) mit mindestens einer Si - OR - Struktureinheit enthalten ist,
(e) mindestens einen Kondensationskatalysator und/oder -vernetzer enthält.

3. Mehrkomponentensystem zur Abdrucknahme nach Anspruch 1 oder 2 mit mindestens zwei Komponenten A und B, **dadurch gekennzeichnet, dass** die Komponente A
(a) mindestens eine Verbindung mit mindestens zwei Alkenylgruppen und
(b) mindestens ein Organohydrogenpolysiloxan,
die Komponente B
(c) mindestens einen Hydrosilylierungskatalysator und
(d2) mindestens eine Verbindung mit mindestens einer Si - OR - Struktureinheit, wobei R = H, Alkyl, Alkoxyalkyl oder Acyl ist,
und die Komponente A oder B
(e) mindestens einen Kondensationskatalysator und/oder -vernetzer enthält.

4. Mehrkomponentensystem zur Abdrucknahme nach einem der Ansprüche 2 bis 3, **dadurch gekennzeichnet, dass** die Komponente A und/oder B
(f) Inhibitoren der Kondensationsreaktionen von Kondensationskatalysatoren und/oder Vernetzern mit Si - OR - Struktureinheiten enthaltenden Verbindungen, wobei R = H, Alkyl, Alkoxyalkyl oder Acyl ist,
(g) wasserabgebende Mittel
(h) Trockenmittel
(i) inerte Trägerstoffe,
(j) Verbindungen zur Reaktionsinhibierung der Hydrosilylierungsreaktion,
(k) verstärkende Füllstoffe,
(l) nicht verstärkende Füllstoffe und/oder
(m) Hilfsstoffe enthält.

5. Mehrkomponentensystem zur Abdrucknahme nach einem der Ansprüche 1 bis 4, **dadurch gekennzeichnet, dass** die Alkinylverbindung (d1)
R¹- C ≡ C - X - R²
ist
und die Si - OR -Verbindung nach (d2) 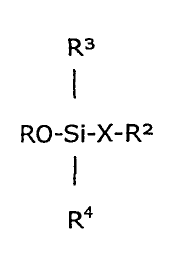 ist, mit
R = H, Alkyl, Alkoxyalkyl oder Acyl;
R¹ = Alkyl-, Aryl-, Arylalkyl, halogensubstituierte Alkyl- und Aryl- gruppen, Cyanalkyl-, Cycloalkyl-, Cycloalkenyl-, insbesondere H-, OH-, Alkoxy-, Acyl-, sowie Kombinationen davon, 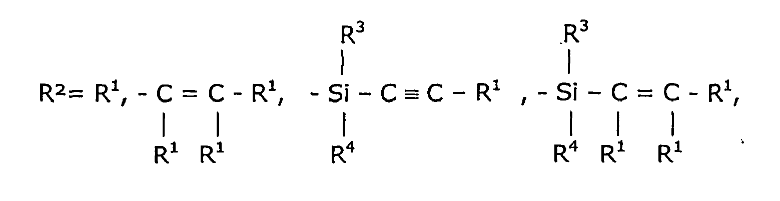 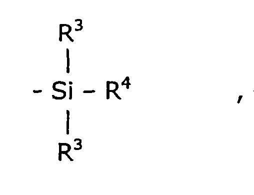 - C ≡ C - R¹ ;
R³ = Alkenyl-, Alkinyl, Halogen-, Aryl-, Alkylaryl-, H-, Halogensubstitu- ierte Alkyl- und Arylgruppen insbesondere Alkyl-, Alkoxy- und Hy- droxyl, sowie Kombinationen davon;
R⁴ = R³, oder R⁴ verschieden von R³, wobei R⁴ insbesondere Alkoxy-, Hydroxyl -, Alkyl-, Methyl-, Alkinyl-, Ethinyl- oder Kombinationen davon; und
X = Polysiloxan, Oligokieselsäureester, Polykiesetsäureester, Polyether, polymere Kohlenwasserstoffe, Polyester und Copolymere der oben ge- nannten Verbindungen ist.

6. Mehrkomponentensystem zur Abdrucknahme nach einem der Ansprüche 1 bis 5, **dadurch gekennzeichnet, dass** die Verbindung nach (d1) oder (d2) 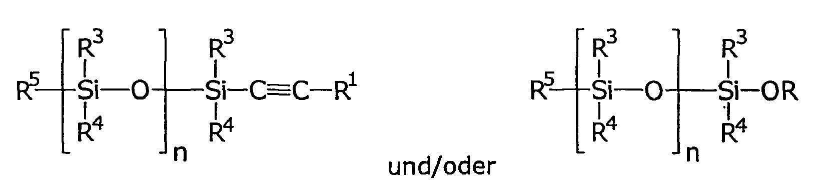 ist,
mit n = 7 - 6000, vorzugsweise 20 bis 6000, insbesondere 100 bis 6000,
wobei R und R¹ bis R⁴ die in Anspruch 4 angegebenen Bedeutungen haben,
R⁵ = -C ≡ C-R¹, H-, Alkyl-, Aryl-, 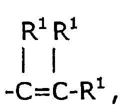 Alkylaryl-, Halogen, -OH,
halogensubstituierte Alkyl- und Arylgruppen, - OR, Aminoal- kyl-, Epoxy-, Cyanalkyl-, Cycloalkyl-, Alkylhydroxyl-, Methacrylat-, Acrylat-, Mercaptoalkyl-, Carboxylat-, Carboxyalkyl- oder Succinicanhydrid ist.

7. Mehrkomponentensystem zur Abdrucknahme nach einem der Ansprüche 1 bis 6, **dadurch gekennzeichnet, dass** die Alkenylverbindung (a) 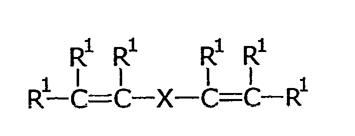 und/oder 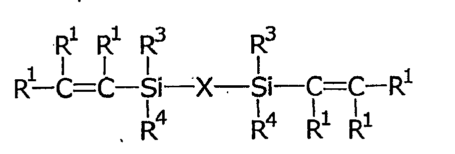 und/oder 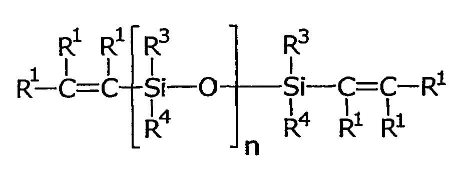 mit n = 0 - 6000
und/oder ein Silan-Dendrimer mit terminalen Alkenylgrupen ist,
wobei die Reste R¹, R², R⁷, R⁴ und X die in den Ansprüchen 4 und 5 angegebenen Bedeutungen haben.

8. Mehrkomponentensystem zur Abdrucknahme nach einem der Ansprüche 1 bis 7, **dadurch gekennzeichnet, dass** die Organohydrogenpolysiloxane (b) Polyalkyl-, Polyaryl- und Polyalkylaryl-, Polyhalogenalkyl-, Polyhalogenaryl- oder Polyhalogenalkylaryl-Siloxane sind, die als Oligomere oder Polymere in linearer, verzweigter oder zyklischer Form oder als QM-Harz vorliegen und mindestens eine Si-H-Bindung aufweisen.

9. Mehrkomponentensystem zur Abdrucknahme nach einem der Ansprüche 1 bis 8, **dadurch gekennzeichnet, dass** die Kondensationskatalysatoren und/oder -vernetzer (e) Aluminiumalkoxide, Antimonalkoxide, Bariumalkoxide, Boralkoxide, Calciumalkoxide, Ceralkoxide, Erbiumalkoxide, Siliciumalkoxide, Galliumalkoxide, Germaniumalkoxide, Hafniumalkoxide, Indiumalkoxide, Eisenalkoxide, Lanthaniumalkoxide, Magnesiumalkoxide, Neodyniumalkoxide, Samariumalkoxide, Strontiumalkoxide, Tantalalkoxide, Titanalkoxide, Zinnalkoxide, Vanadiumalkoxide, Yttriumalkoxide, Zinkalkoxide, Zirconiumalkoxide, Titan- oder Zirkoniumverbindungen, insbesondere Titan-, Zirconium- und Hafniumalkoxide, sowie Doppelmetallalkoxide, Chelate und Oligo- und Polykondensate der o.g. Alkoxide, Dialkylzinndiacetat, Zinn(II)octoat, Dialkylzinndiacylat oder Dialkylzinnoxid sind.

10. Mehrkomponentensystem zur Abdrucknahme nach einem der Ansprüche 1 bis 9, **dadurch gekennzeichnet, dass** die Hydrosilylierungskatalysatoren (c) Übergangsmetalle der 8. Nebengruppe, vorzugsweise Platin, Palladium und Rhodium oder deren Salze, Komplexe und Kolloide, vorzugsweise Platinkomplexe und Salze der Hexachloroplatinsäure sind.

11. Mehrkomponentensystem zur Abdrucknahme nach einem der Ansprüche 4 bis 10, **dadurch gekennzeichnet, dass** die Inhibitoren der Kondensationsreaktionen (f)
Di-, Tri-, Oligo- und Polydialkylsiloxane der allgemeinen Formel
Z - SiR₂ - O - (SiR₂O)ₙ - SiR₃
oder
Z - SiR₂ - O- (SiR₂O)ₙ - SiR₂ - Z
sind, wobei Z OH oder NR₂ ist, R gleiche oder verschiedene gegebenenfalls substituierte Kohlenwasserstoffreste wie Alkyl-, Alkenyl-, Aryl- oder Alkinylsind und n= 0 oder eine ganze Zahl von 1 bis 100 ist; oder
aliphatische Diole, Diamine, Diphosphane, Polyamine, Polyphosphane oder Polyole, OH-, NH- oder PR-funktionelle Polyether oder andere chelatisierende Verbindungen sind.

12. Mehrkomponentensystem zur Abdrucknahme nach einem der Ansprüche 4 bis 11, **dadurch gekennzeichnet, dass** die wasserabgebenden Mittel (g) anorganische Füllstoffe, die eine oberflächlich gebundene Restfeuchtigkeit oder im Kristallgitter gebundenes Wasser enthalten, Zeolithe, gezielt befeuchtete Füllstoffe oder organische Stoffe mit definiertem Wassergehalt sind.

13. Mehrkomponentensystem zur Abdrucknahme nach einem der Ansprüche 4 bis 12, **dadurch gekennzeichnet, dass** die Trockenmittel (h) Zeolithe, getrocknete Füllstoffe oder wasseraufnehmende organische Verbindungen wie Oxazolidine und Alkalisalze der Poly(meth)acrylsäure (Superabsorber) eingesetzt sind.

14. Mehrkomponentensystem zur Abdrucknahme nach einem der Ansprüche 4 bis 13, **dadurch gekennzeichnet, dass** die inerten Trägerstoffe (i) Mineralöle, verzweigte Kohlenwasserstoffe, Vaseline, Ester, Phtalsäureester, Acetyltributylcitrat, Polyalkylenoxide und Polyester und deren Copolymere sind.

15. Mehrkomponentensystem zur Abdrucknahme nach einem der Ansprüche 4 bis 14, **dadurch gekennzeichnet, dass** die Verbindungen zur Reaktionsinhibierung der Hydrosilylierungsreaktion (j) kurzkettige Organopolysiloxane der allgemeinen Formel
CH₂ = CH - SiR₂O - (SiR₂O)ₙ - SiR₂ - CH = CH₂,
sind,
wobei R gleiche oder verschiedene, gegebenenfalls substituierte Kohlenwasserstoffreste wie Alkyl-, Alkenyl-, Aryl-, Alkinyl-, Alkenyl-, sowie Alkinyl-terminierte Siloxanreste sind, und
n = 0 oder eine ganze Zahl von 1 bis 6 ist;
oder vinylhaltige, cyclische Siloxane, wie beispielweise Tetravinyltetramethylcyclotetrasiloxan oder endständige Doppel- oder Dreifachbindungen enthaltende, organische Hydroxyl-Verbindungen, Diethylmaleat, Alkylsilan, Arylsilan, Alkenylsilan, Alkinylsilan, Benzotriazol, Verbindungen mit 1,4-Enin-Struktureinheit, Verbindungen mit 1,3-Enin-Struktureinheit wie 2-Methyl-1-hexen-3-yne, Ethyi-3-(trimethylsilyl) propynoat, Bis-(phenylethinyl)dimethylsilan, Diyne wie Decadiyne oder Dodecadiyne, Polyyne, Diene, Polyene wie Decatrien, (1,3-Dioxan-2-yl-ethinyl)trialkylsilan, 1,4-Divinyltetramethyldisilylethan, Amine oder Phosphane sind.

16. Mehrkomponentensystem zur Abdrucknahme nach einem der Ansprüche 4 bis 15, **dadurch gekennzeichnet, dass** die verstärkenden Füllstoffe (k) hochdisperse, aktive Füllstoffe wie Titandioxid, Aluminiumoxid, Zinkoxid, vorzugsweise naßgefällte oder pyrogen gewonnene Kieselsäure sind, die gegebenenfalls hydrophil oder hydrophobiert vorliegen; oder mineralische, faserförmige Füllstoffe, wie Wollastonit, oder synthetische, faserförmige Füllstoffe wie Glasfasern, Keramikfasern oder Kunststofffasern sind.

17. Mehrkomponentensystem zur Abdrucknahme nach einem der Ansprüche 4 bis 16, **dadurch gekennzeichnet, dass** die nicht verstärkende Füllstoffe (I) Metalloxide, Metalloxidhydroxide, Mischoxide oder Mischhydroxide, vorzugsweise Siliciumdioxid, insbesondere in Form von Quarz und seinen kristallinen Modifikationen, Quarzgut sowie Aluminiumoxid, Calciumoxid, Aluminiumhydroxid, sowie Calciumcarbonat, Kieselgur, Diatomeenerde, Talkum, gemahlene Gläser und Füllstoffe auf Kunststoff-Basis, beispielsweise Polymethylmethacrylat, Polycarbonat, Polyvinylchlorid, Siliconharzpulver oder Pulver auf Basis fluororganischer Verbindungen sind, wobei die nicht verstärkenden Füllstoffe gegebenenfalls oberflächenbehandelt (gecoated) sind.

18. Mehrkomponentensystem zur Abdrucknahme nach einem der Ansprüche 4 bis 17, **dadurch gekennzeichnet, dass** die Hilfsstoffe (m) Farbstoffe, Tenside, Opaker, Mattierungsmittel, z.B. Titandioxid oder Zinkoxid, Weichmacher, Wasserstoff-Adsorber/Absorber, radioopake Substanzen oder siliziumorganische MQ-Harze mit Si-Vinyl, Si-OR, Si-Ethinyl- und/oder SiH-Gruppen oder Verbindungen oder Puffer und Stoffe zur Einstellung des pH-Bereichs sind.

19. Mischungen, erhältlich durch Mischen der Komponenten A und B der Ansprüche 2 bis 18.

20. Mischungen nach Anspruch 19, **dadurch gekennzeichnet, dass** während und nach dem Mischen der Komponenten die Mischung in einer ersten Stufe von einer dünneren mischergängigen Ausgangskonsistenz zu einer zäheren, plastischen Phase übergeht, in der das Material bei der Zahnabformung im Abformlöffel einen hohen Stempeldruck aufbaut und in einer zweiten Stufe zu seiner endgültigen elastischen Form aushärtet.

21. Mischungen nach Anspruch 19, **dadurch gekennzeichnet dass** die Mischungen ausgehärtet sind.

22. Mischungen nach Anspruch 19, **dadurch gekennzeichnet, dass** die Mischungen zu Beginn des Mischens in einem ersten Zustand eine mischergängige Konsistenz von > 26 mm, bevorzugt > 30 mm (nach ISO 4823) aufweist, wonach die Mischungen durch Kondensationsreaktionen von SiOR-Gruppen und/oder durch Hydrosilylierungsreaktionen von Alkinylgruppen mit SiH-Gruppen in einen zäheren zweiten Zustand mit einer Konsistenz von < 35 mm, bevorzugt < 30 mm (nach ISO 4823) übergehen, wobei diese Konsistenz über einen Zeitraum von mindestens 15 sec erhalten bleibt, und die Mischungen danach durch eine Hydrosilylierungsreaktion von Alkenylgruppen mit SiH-Gruppen nach Aushärten in einen dritten festen, elastischen Zustand übergeht.

23. Verwendung eines Mehrkomponentensystems nach einem der Ansprüche 1 bis 18 zur Herstellung eines Abdrucks von einem abzuformenden Gegenstand, wobei die Abformmasse durch Mischen der Komponenten hergestellt wird, wobei die Abformmasse zunächst aus einem Behälter in einem ersten Zustand austragbar ist, danach in einen zweiten Zustand übergeht, in dem die Viskosität der Abformmasse erhöht ist, wonach ein Abdruck von einem abzuformenden Gegenstand genommen wird, und die Abformmasse danach in einen dritten, festen Zustand übergeht, in dem ein Abformergebnis festgehalten ist, wobei der zweite Zustand durch abgestufte Hydrosilylierungsreaktionen zwischen Alkinyl- und Alkenyl-Struktureinheiten mit Si-H Gruppen enthaltenden Verbindungen und/oder durch abgestufte Additionsreaktion (zwischen Alkenyl und SiH-Gruppen) und Kondensationsreaktion (von SiOR-Gruppen mit Kondensationskatalysatoren) eingestellt wird.

24. Verwendung nach Anspruch 23, **dadurch gekennzeichnet, dass** die Konsistenz der Abformmasse zu Beginn des Mischens im ersten Zustand > 26mm, bevorzugt > 30 mm ist (nach ISO 4823) und die Abformmasse in diesem ersten Zustand mischergängig ist, dass die Konsistenz der Abformmasse im zweiten Zustand < 35 mm, bevorzugt < 30 mm (nach ISO 4823) ist, wobei die Abformmasse zäher ist als im ersten Zustand, und dass die Abformmasse in diesem zweiten Zustand bis zum Ende der Gesamtverarbeitungszeit mindestens 15 sec, erhalten bleibt.

25. Mischungen nach Anspruch 19, **dadurch gekennzeichnet, dass** die Mischungen zu Beginn des Mischens in einem ersten Zustand eine mischergängige Konsistenz von > 26 mm, bevorzugt > 30 mm (nach ISO 4823) aufweist, wonach die Mischungen durch eine Hydrosilylierungsreaktion von Alkenylgruppen mit SiH-Gruppen in einen zäheren zweiten Zustand mit einer Konsistenz von < 35 mm, bevorzugt < 30 mm (nach ISO 4823) übergehen, wobei diese Konsistenz über einen Zeitraum von mindestens 15 sec erhalten bleibt, und die Mischungen danach durch Kondensationsreaktionen von SiOR-Gruppen und/oder durch Hydrosilylierungsreaktionen von Alkinylgruppen mit SiH-Gruppen in einen dritten festen, elastischen Zustand übergeht.

## Claims

1. A multi-component system for impression taking comprising
(a) at least one compound having at least two alkenyl groups;
(b) at least one organohydrogenpolysiloxane; and
(c) at least one hydrosilylation catalyst,
**characterized in that** it comprises
(d1) at least one polymeric compound having at least one alkynyl group and/or
(d2) at least one compound having at least one Si - OR structural unit, R being H, alkyl, alkoxyalkyl, or acyl; and
if a compound (d2) having at least one Si - OR structural unit is present,
(e) at least one condensation catalyst and/or condensation crosslinking agent.

2. A multi-component system for impression taking according to claim 1 having at least two components A and B, **characterized in that** component A comprises
(a) at least one compound having at least two alkenyl groups and
(b) at least one organohydrogenpolysiloxane;
and
(d1) at least one polymeric compound having at least one alkynyl group and/or
(d2) at least one compound having at least one Si - OR structural unit, R being H, alkyl, alkoxyalkyl, or acyl;
component B comprises
(c) at least one hydrosilylation catalyst; and
component A and/or B comprises, if a compound (d2) having at least one Si - OR structural unit is present,
(e) at least one condensation catalyst and/or condensation crosslinking agent.

3. A multi-component system for impression taking according to claim 1 or claim 2 having at least two components A and B, **characterized in that** component A comprises
(a) at least one compound having at least two alkenyl groups and
(b) at least one organohydrogenpolysiloxane;
component B comprises
(c) at least one hydrosilylation catalyst and
(d2) at least one compound having at least one Si - OR structural unit, R being H, alkyl, alkoxyalkyl, or acyl;
and component A or B comprises
(e) at least one condensation catalyst and/or condensation crosslinking agent.

4. A multi-component system for impression taking according to any one of claims 2 to 3, **characterized in that** component A and/or B comprises
(f) inhibitors of the condensation reactions of condensation catalysts and/or condensation crosslinking agents with compounds comprising Si - OR structural units, R being H, alkyl, alkoxyalkyl, or acyl;
(g) water-liberating agents;
(h) drying agents;
(i) inert carrier substances;
(j) compounds for inhibiting the hydrosilylation reaction;
(k) reinforcing fillers;
(l) non-reinforcing fillers; and/or
(m) auxiliary agents.

5. A multi-component system for impression taking according to any one of claims 1 to 4, **characterized in that** the alkynyl compound (d1) is
R¹-C≡C-X-R²
and the Si - OR compound of (d2) is 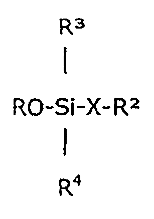 wherein
R = H, alkyl, alkoxyalkyl, or acyl;
R¹ = alkyl, aryl, arylalkyl, halogen-substituted alkyl and aryl groups, cyanoalkyl, cycloalkyl, cycloalkenyl, in particular H, OH, alkoxy, acyl, and combinations thereof, 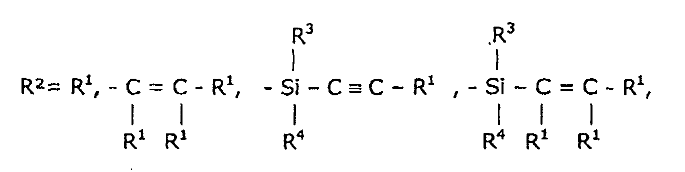 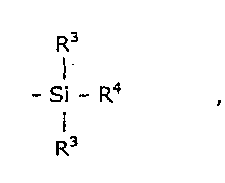 - C≡C - R¹;
R³ = alkenyl, alkynyl, halogen, aryl, alkylaryl, H, halogen-substituted alkyl and aryl groups, in particular alkyl, alkoxy, and hydroxyl, and combinations thereof;
R⁴ = R³ , or R⁴ is different from R³ , R⁴ being in particular alkoxy, hydroxyl, alkyl, methyl, alkynyl, ethynyl, or combinations thereof; and
X = polysiloxane, oligosilicic acid esters, polysilicic acid esters, polyethers, polymeric hydrocarbons, polyesters and copolymers of the compounds mentioned above.

6. A multi-component system for impression taking according to any one of claims 1 to 5, **characterized in that** the compound of (d1) or (d2) is 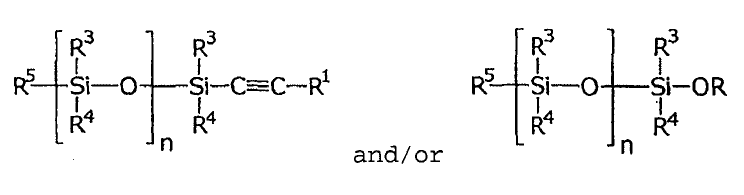 wherein n = 7 - 6000, preferably 20 to 6000, most preferably 100 to 6000,
R and R¹ to R⁴ having the meanings given in claim 5;
R⁵ = -C≡C-R¹, H, alkyl, aryl, 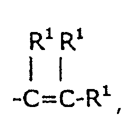 alkylaryl, halogen, OH, halogen-substituted alkyl and aryl groups, OR, aminoalkyl, epoxy, cyanoalkyl, cycloalkyl, alkylhydroxyl, methacrylate, acrylate, mercaptoalkyl, carboxylate, carboxyalkyl, or succinic anhydride.

7. A multi-component system for impression taking according to any one of claims 1 to 6, **characterized in that** the alkenyl compound (a) is 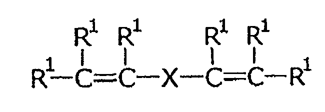 and/or 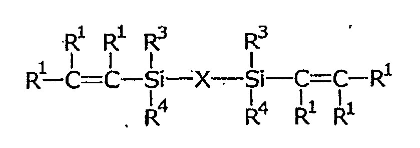 and/or 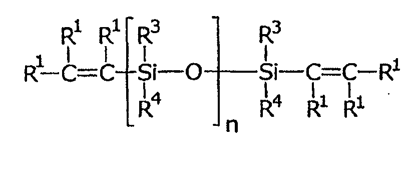 wherein n = 0 - 6000 and/or a silane dendrimer having terminal alkenyl groups,
the radicals R¹, R², R³ , R⁴ , and X having the meanings given in claim 5.

8. A multi-component system for impression taking according to any one of claims 1 to 7, **characterized in that** the organohydrogenpolysiloxanes (b) are polyalkyl-, polyaryl- and polyalkylaryl-, polyhalogenalkyl-, polyhalogenaryl-, or polyhalogenalkylaryl-siloxanes, which are present as oligomers or polymers in linear, branched, or cyclic form or as QM resin and have at least one Si-H bond.

9. A multi-component system for impression taking according to any one of claims 1 to 8, **characterized in that** the condensation catalysts and/or condensation crosslinking agents (e) are aluminum alkoxides, antimony alkoxides, barium alkoxides, boron alkoxides, calcium alkoxides, cerium alkoxides, erbium alkoxides, silicon alkoxides, gallium alkoxides, germanium alkoxides, hafnium alkoxides, indium alkoxides, iron alkoxides, lanthanum alkoxides, magnesium alkoxides, neodymium alkoxides, samarium alkoxides, strontium alkoxides, tantalum alkoxides, titanium alkoxides, tin alkoxides, vanadium alkoxides, yttrium alkoxides, zinc alkoxides, zirconium alkoxides, titanium or zirconium compounds, in particular titanium, zirconium, and hafnium alkoxides, and two metal alkoxides, chelates and oligo- and polycondensates of the alkoxides mentioned above, dialkyltin diacetate, tin(II) octoate, dialkyltin diacylate, or dialkyltin oxide.

10. A multi-component system for impression taking according to any one of claims 1 to 9, **characterized in that** the hydrosilylation catalysts (c) are transition metals of the 8^{th} subgroup, preferably platinum, palladium, and rhodium, or salts, complexes, and colloids thereof, preferably platinum complexes and salts of hexachloroplatinic acid.

11. A multi-component system for impression taking according to any one of claims 4 to 10, **characterized in that** the inhibitors of the condensation reactions (f) are di-, tri-, oligo-, and polydialkylsiloxanes of the general formula
Z - SiR₂-O(SiR₂O)ₙ-SiR₃
or
Z - SiR₂-O-(SiR₂O)ₙ-SiR₂-Z
wherein Z is OH or NR₂, R are the same or different, optionally substituted hydrocarbon radicals such as alkyl, alkenyl, aryl, or alkynyl, and n = 0 or an integer from 1 to 100; or
aliphatic diols, diamines, diphosphanes, polyamines, polyphosphanes, or polyols, OH-, NH-, PR-functional polyethers, or other chelating compounds.

12. A multi-component system for impression taking according to any one of claims 4 to 11, **characterized in that** the water-liberating agents (g) are inorganic fillers comprising surface-bound residual moisture or water bound in the crystal lattice, zeolites, selectively moistened fillers, or organic substances having a defined water content.

13. A multi-component system for impression taking according to any one of claims 4 to 12, **characterized in that** the drying agents (h) used are zeolites, dried fillers, or water-absorbing organic compounds such as oxazolidines and alkaline salts of poly(meth)acrylic acid (superabsorbents).

14. A multi-component system for impression taking according to any one of claims 4 to 13, **characterized in that** the inert carrier substances (i) are mineral oils, branched hydrocarbons, paraffin jelly, esters, phthalic acid esters, acetyl tributyl citrate, polyalkylene oxides, and polyesters and copolymers thereof.

15. A multi-component system for impression taking according to any one of claims 4 to 14, **characterized in that** the compounds for inhibiting the hydrosilylation reaction (j) are short-chain organopolysiloxanes of the general formula
CH₂ = CH - SiR₂O - (SiR₂O)ₙ - SiR₂ - CH = CH₂,
wherein R are the same or different, optionally substituted hydrocarbon radicals such as alkyl-, alkenyl-, aryl-, alkynyl-, alkenyl-, and alkynyl-terminated siloxane radicals, and
n = 0 or an integer from 1 to 6;
or vinyl-containing, cyclic siloxanes, such as for example tetravinyltetramethylcyclotetrasiloxane or terminal double or triple bond-containing, organic hydroxyl compounds, diethyl maleate, alkylsilane, arylsilane, alkenylsilane, alkynylsilane, benzotriazole, compounds having a 1,4-eneyne structural unit, compounds having a 1,3-eneyne structural unit, such as 2-methyl-1-hexene-3-ynes, ethyl-3-(trimethylsilyl) propynoate, bis-(phenylethynyl)dimethylsilane, diynes, such as decadiynes or dodecadiynes, polyynes, dienes, polyenes, such as decatriene, (1,3-dioxane-2-yl-ethynyl)trialkylsilane, 1,4-divinyltetramethyldisilylethane, amines, or phosphanes.

16. A multi-component system for impression taking according to any one of claims 4 to 15, **characterized in that** the reinforcing fillers (k) are highly dispersed, active fillers, such as titanium dioxide, aluminum oxide, zinc oxide, preferably wet-precipitated or pyrogenically obtained silica, which are optionally present in hydrophilic or hydrophobed form; or mineral, fiber-like fillers, such as wollastonite, or synthetic, fiber-like fillers, such as glass fibers, ceramic fibers, or plastic fibers.

17. A multi-component system for impression taking according to any one of claims 4 to 16, **characterized in that** the non-reinforcing fillers (l) are metal oxides, metal oxide hydroxides, mixed oxides or mixed hydroxides, preferably silicon dioxide, in particular in the form of quartz and its crystalline modifications, fused silica and aluminum oxide, calcium oxide, aluminum hydroxide, and calcium carbonate, kieselguhr, diatomaceous earth, talcum, milled glasses, and fillers based on plastics, for example polymethylmethacrylate, polycarbonate, polyvinyl chloride, silicone resin powders, or powders based on fluoroorganic compounds, the non-reinforcing fillers optionally being surface-treated (coated).

18. A multi-component system for impression taking according to any one of claims 4 to 17, **characterized in that** the auxiliary agents (m) are dyes, surfactants, opaque-making agents, matting agents, e.g. titanium dioxide or zinc oxide, plasticizers, hydrogen adsorbers/absorbers, radio-opaque substances, or organosilicon MQ resins having Si-vinyl, Si-OR, Si-ethynyl and/or SiH groups, or compounds or buffers and substances for adjusting the pH range.

19. Mixtures obtainable by mixing components A and B of claims 2 to 18.

20. Mixtures according to claim 19, **characterized in that** during and after mixing of the components, in a first stage, the mixture changes from a thinner starting consistency that can be passed through a mixer to a more viscous, plastic phase, in which the material builds up a high pressure in the impression tray when the impression of the teeth is taken, and, in a second stage, hardens to its final elastic form.

21. Mixtures according to claim 19, **characterized in that** the mixtures are hardened.

22. Mixtures according to claim 19, **characterized in that** the mixtures have at the beginning of the mixing in a first state a consistency that can be passed through a mixer of > 26 mm, preferably > 30 mm (in accordance with ISO 4823), whereupon by condensation reactions of SiOR groups and/or by hydrosilylation reactions of alkynyl groups with SiH groups, the mixtures change into a more viscous second state having a consistency of < 35 mm, preferably < 30 mm (in accordance with ISO 4823), this consistency being maintained for a period of time of at least 15 sec., and afterwards the mixtures change by means of a hydrosilylation reaction of alkenyl groups with SiH groups after hardening into a third solid, elastic state.

23. The use of a multi-component system according to any one of claims 1 to 18 for making an impression of an object to be molded, where the impression material is prepared by mixing the components, and where the impression material initially is dischargeable from a container in a first state, afterwards changes into a second state, in which the viscosity of the impression material is increased, whereupon an impression of an object to be molded is taken, and afterwards the impression material changes into a third, solid state, in which an impression result is retained, the second state being adjusted by gradual hydrosilylation reactions between alkynyl and alkenyl structural units with SiH group-containing compounds and/or by gradual addition reaction (between alkenyl and SiH groups) and condensation reaction (of SiOR groups with condensation catalysts).

24. The use according to claim 23, **characterized in that** at the beginning of mixing, the consistency of the impression material is in the first state > 26 mm, preferably > 30 mm (in accordance with ISO 4823) and the impression material can be passed through a mixer in this first state, the consistency of the impression material is in the second state < 35 mm, preferably < 30 mm (in accordance with ISO 4823), the impression material being more viscous than in the first state, and the impression material is maintained in this second state until the end of the total pot life for at least 15 sec.

25. Mixtures according to claim 19, **characterized in that** at the beginning of mixing, the mixtures have in a first state a consistency that can be passed through a mixer of > 26 mm, preferably > 30 mm (in accordance with ISO 4823), whereupon by a hydrosilylation reaction of alkenyl groups with SiH groups, the mixtures change into a more viscous second state having a consistency of < 35 mm, preferably < 30 mm (in accordance with ISO 4823), this consistency being maintained for a period of time of at least 15 sec., and afterwards the mixtures change by means of condensation reactions of SiOR groups and/or by hydrosilylation reactions of alkynyl groups with SiH groups into a third solid, elastic state.

## Revendications

1. Système à plusieurs composants destiné à la réalisation d'empreintes, contenant
(a) au moins un composé renfermant au moins deux groupes alcényle,
(b) au moins un polysiloxane organohydrogéné et
(c) au moins un catalyseur d'hydrosilylation,
**caractérisé en ce qu'**il contient
(d1) au moins un composé polymère refermant au moins un groupe alcynyle et/ou
(d2) au moins un composé renfermant au moins une unité structurelle Si - OR, R étant H, alkyle, alkoxyalkyle ou acyle, et,
s'il contient un composé (d2) renfermant au moins une unité structurelle Si - OR,
(e) au moins un catalyseur et/ou agent de réticulation pour des réactions de condensation.

2. Système à plusieurs composants destiné à la réalisation d'empreintes selon la revendication 1 et renfermant au moins deux composants A et B, **caractérisé en ce que** le composant A contient
(a) au moins un composé renfermant au moins deux groupes alcényle et
(b) au moins un polysiloxane organohydrogéné ainsi que
(d1) au moins un composé polymères refermant au moins un groupe alcynyle et/ou
(d2) au moins un composé renfermant au moins une unité structurelle Si - OR, R étant H, alkyle, alkoxyalkyle ou acyle,
et que le composant B contient
(c) au moins un catalyseur d'hydrosilylation
et que les composants A et/ou B contiennent, si le système contient un composé (d2) renfermant au moins une unité structurelle Si - OR,
(e) au moins un catalyseur et/ou agent de réticulation pour des réactions de condensation.

3. Système à plusieurs composants destiné à la réalisation d'empreintes selon les revendications 1 ou 2 et renfermant au moins deux composants A et B, **caractérisé en ce que** le composant A contient
(a) au moins un composé renfermant au moins deux groupes alcényle et
(b) au moins un polysiloxane organohydrogéné,
et que le composant B contient
(c) au moins un catalyseur d'hydrosilylation et
(d2) au moins un composé renfermant au moins une unité structurelle Si - OR, R étant H, alkyle, alkoxyalkyle ou acyle,
et que le composant A ou B contient
(e) au moins un catalyseur et/ou agent de réticulation pour des réactions de condensation.

4. Système à plusieurs composants destiné à la réalisation d'empreintes selon l'une des revendications 2 à 3, **caractérisé en ce que** le(s) composant(s( A et/ou B contient/contiennent
(f) des agents inhibiteurs des réactions de condensation de catalyseurs et/ou agents de réticulation pour des réactions de condensation avec des composés contenant des unités structurelles Si - OR, R étant H, alkyle, alkoxyalkyle ou acyle,
(g) des agents dégageant de l'eau
(h) des agents desséchants
(i) des matériaux de support inertes,
(j) des composés destinés à inhiber la réaction d'hydrosilylation,
(k) des charges renforçantes,
(1) des charges non renforçantes et/ou
(m) des agents auxiliaires.

5. Système à plusieurs composants destiné à la réalisation d'empreintes selon l'une des revendications 1 à 4, **caractérisé en ce que** le composé d'alcynyle (d1)
est le R¹ - C≡C - X - R²
et que le compose Si - OR selon (d2) est le 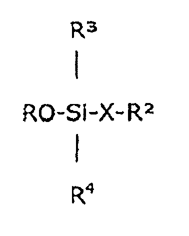 dans laquelle
R = H, alkyle, alkoxyalkyle ou acyle;
R¹ = alkyle, aryle, Arylalkyle, des groupes alkyle et aryle substitués avec des halogènes, cyanoalkyle, cycloalkyle, cycloalcényle, notamment H, OH, alkoxy, acyle ainsi que des combinaisons desdits groupes, 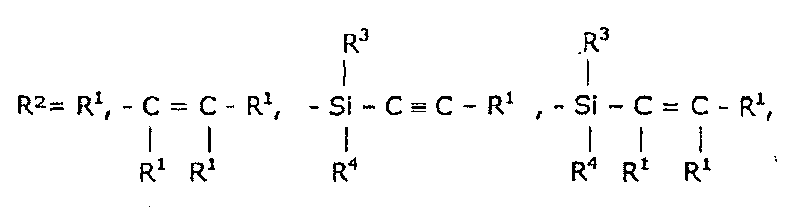 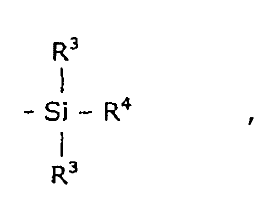 -C≡C-R¹ ;
R³ = alcényle, alcynyle, halogène, aryle, alkylaryle, H, groupes alkyle et aryle substitués avec des halogènes, notamment alkyle, alkoxy et hydroxyle, ainsi que des combinaisons desdits groupes,
R⁴ = R³ , ou R⁴ différent de R³ , R⁴ étant notamment alkoxy, hydroxyle, alkyle, méthyle, alcynyle, ethynyle ou des combinaisons desdits groupes; ou
X = polysiloxane, oligoesters d'acide silicique, polyesters d'acide silicique, polyéthers, hydrocarbures polymères, polyester ou copolymères des desdits composés.

6. Système à plusieurs composants destiné à la réalisation d'empreintes selon l'une des revendications 1 à 5, **caractérisé en ce que** le composé selon (d1) ou (d2) est 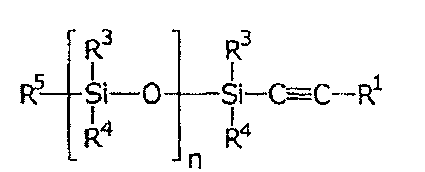 et/ou 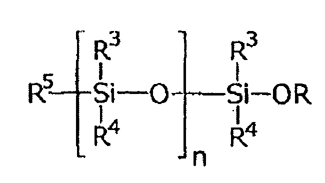 n étant compris entre 7 et 6000, de préférence entre 20 et 6000 et notamment entre 100 et 6000,
R et R¹ à R⁴ ayant la signification indiquée dans la revendication 5,
R⁵ étant -C≡C-R¹ , H, alkyle, aryle, 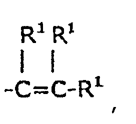 alkylaryle, halogène, OH, groupes alkyle et aryle substitués avec des halogènes, OR, aminoalkyle, époxy, cyanoalkyle, alkylhydroxyle, méthacrylate, acrylate, mercaptoalkyle, carboxylate, carboxyalkyle ou anhydride succinique.

7. Système à plusieurs composants destiné à la réalisation d'empreintes selon l'une des revendications 1 à 6, **caractérisé en ce que** le composé d'alcényle (a) est 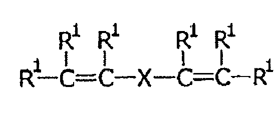 et/ou 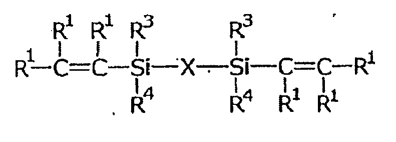 et/ou 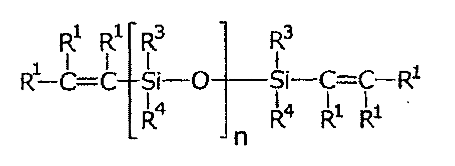 n étant compris entre 0 et 6000,
et/ou un dendrimère de silane pourvu de groupes alcényle terminaux,
les restes R¹, R², R³, R⁴ et X ayant les significations indiquées dans la revendication 5.

8. Système à plusieurs composants destiné à la réalisation d'empreintes selon l'une des revendications 1 à 7, **caractérisé en ce que** les polysiloxanes organohydrogénés (b) sont des siloxanes pourvus de groupes polyalkyle, polyaryle et polyalkylaryle, polyalkyle halogéné, polyaryle halogéné ou polyalkylaryle halogéné et qu'ils se présentent sous forme d'oligomères ou de polymères à structure linéaire, ramifiée ou cyclique ou sous forme d'une résine QM et qu'ils comportent au moins une liaison Si-H.

9. Système à plusieurs composants destiné à la réalisation d'empreintes selon l'une des revendications 1 à 8, **caractérisé en ce que** les catalyseurs et/ou agents de réticulation pour des réactions de condensation (e) sont des alkoxydes d'aluminium, alkoxydes d'antimoine, alkoxydes de baryum, alkoxydes de bore, alkoxydes de calcium, alkoxydes de cérium, alkoxydes d'erbium, alkoxydes de silicium, alkoxydes de gallium, alkoxydes de germanium, alkoxydes d'hafnium, alkoxydes d'indium, alkoxydes de fer, alkoxydes de lanthane, alkoxydes de magnésium, alkoxydes de néodyme, alkoxydes de samarium, alkoxydes de strontium, alkoxydes de tantale, alkoxydes de titane, alkoxydes d'étain, alkoxydes de vanadium, alkoxydes d'yttrium, alkoxydes de zinc, des composés de titane ou de zirconium, notamment des alkoxydes de titane, de zirconium et d'hafnium ou des alkoxydes mixtes de deux métaux, des chélates ou oligo- et polycondensats desdits alkoxydes, de l'acétate de dialkylétain, de l'octoate d'étain (II), du diacylate de dialkylétain ou du dialkyloxyde d'étain.

10. Système à plusieurs composants destiné à la réalisation d'empreintes selon l'une des revendications 1 à 9, **caractérisé en ce que** les catalyseurs d'hydrosilylation (c) sont des métaux de transition issu du groupe VIII, s'agissant préférentiellement de platine, de palladium et de rhodium ou de leurs sels, complexes et colloides, notamment de complexes de platine et de sels de l'acide hexachloroplatinique.

11. Système à plusieurs composants destiné à la réalisation d'empreintes selon l'une des revendications 4 à 10, **caractérisé en ce que** les agents inhibiteurs de réactions de condensation (f) sont des di-, tri-, oligo ou polydialkylsiloxanes répondant à la formule générale
Z - SiR₂ - O - (SiR₂O)ₙ - SiR₃
ou
Z - SiR₂ - O - (SiR₂O)ₙ - SiR₂ - Z
Z étant OH ou NR₂ et R représentant des restes d'hydrocarbures identiques et différents, le cas échéant substitués, comme alkyle, alcényle, aryle ou alcynyle et n étant compris entre 0 et un nombre entier compris entre 1 et 100; ou
des diols diamines, diphosphanes, polyamines, polyphosphanes ou polyols aliphatiques, des polyéther pourvus de groupes fonctionnels OH, NH ou PR ou d'autres composés chélatants.

12. Système à plusieurs composants destiné à la réalisation d'empreintes selon l'une des revendications 4 à 11, **caractérisé en ce que** les agents dégageant de l'eau (g) sont des charges inorganique contenant de l'humidité résiduelle retenue sur leur surface ou de l'eau intégrée à leur réseau cristallin, des zéolithes, des charges volontairement humidifiées ou des matières organiques avec une teneur en eau définie.

13. Système à plusieurs composants destiné à la réalisation d'empreintes selon l'une des revendications 4 à 12, **caractérisé en ce que** les agents desséchants (g) mis en oeuvre sont des charges séchées ou des composés organiques absorbant de l'eau comme les oxazolidines et les sels alcalins de l'acide poly(méth)acrylique (appelés superabsorbants).

14. Système à plusieurs composants destiné à la réalisation d'empreintes selon l'une des revendications 4 à 13, **caractérisé en ce que** les matériaux de support inertes (i) sont des huiles minérales, des hydrocarbures ramifiés, de la vaseline, des esters, des esters d'acide phtalique, du tributylcitrate d'acétyle, des polyoxydes d'alkylène et des polyesters ainsi que leurs copolymères.

15. Système à plusieurs composants destiné à la réalisation d'empreintes selon l'une des revendications 4 à 14, **caractérisé en ce que** les composés destinés à inhiber la réaction d'hydrosilylation (j) sont des organopolysiloxanes à courte chaîne répondant à la formule générale
CH₂ = CH - SiR₂O - (SiR₂O)ₙ - SiR₂ - CH = CH₂,
dans laquelle R représente des restes d'hydrocarbures identiques ou différents, le cas échéant substitués, comme des restes de siloxanes pourvus de groupes terminaux alkyle, alcényle, aryle, alcynyle ou bien alcényle,
n étant 0 ou un nombre entier comprise entre 1 et 6;
ou des siloxanes cycliques contenant du vinyle, comme par exemple le tétravinyltétraméthylcyclotétrasiloxane, ou des composés organiques hydroxylés contenant des liaisons double ou triple en position terminale, du maléate de diéthyle, de l'alkylsilane, de l'arylsilane, de l'alkénylsilane, de l'alkynylsilane, du benzotriazole, des composés pourvus d'une unité structurelle de type 1,4-énine, des composés pourvus d'une unité structurelle de type 1,3-énine comme les 2-méthyl-1-hexèn-3-ines, du 3-(triméthylsilyl)propinoate d'éthyle, du bis(phénylethynyl)diméthylsilane, des diynes comme le décadiyne ou le dodécadiyne, des polyynes, des diènes, des polyènes comme le décatriène, le (1,3-dioxane-2-yl-éthynyl)trialkylsilane, le 1,4-divinyltétraméthyldisilyléthane, des amines ou des phosphanes.

16. Système à plusieurs composants destiné à la réalisation d'empreintes selon l'une des revendications 4 à 15, **caractérisé en ce que** les charges renforçantes (k) sont des charges actives et hautement dispersées comme le dioxyde de titane, l'oxyde d'aluminium, l'oxyde de zinc, s'agissant préférentiellement d'acide silicique précipité d'une solution ou pyrogéné, se présentant le cas échéant sous leurs formes hydrophile ou hydrophobée; ou des charges minérales fibreuses comme la wollastonite ou de charges synthétiques fibreuses comme les fibres de verre, les fibres de céramique ou les fibres en matière plastique.

17. Système à plusieurs composants destiné à la réalisation d'empreintes selon l'une des revendications 4 à 16, **caractérisé en ce que** les charges non-renfonçantes (1) sont des oxydes métalliques, des hydroxydes d'oxydes métalliques, des oxydes mixtes ou des hydroxydes mixtes, s'agissant préférentiellement de dioxyde de silicium, notamment sous forme de quartz et de ses modifications cristallines, du verre quartz ainsi que de l'oxyde d'aluminium, de l'oxyde de calcium, de l'hydroxyde d'aluminium ainsi que du carbonate de calcium, du kieselguhr, de la terre de diatomées, du talc, des verres broyés et des charges à base de matières plastiques, par exemple de polyméthylméthacrylate, de polycarbonate, de polychlorure de vinyle, de résine silicone en poudre ou de poudre à base de composés organiques fluorés, les charges non-renforçantes pouvant le cas échéant avoir subi un traitement de surface (coating).

18. Système à plusieurs composants destiné à la réalisation d'empreintes selon l'une des revendications 4 à 17, **caractérisé en ce que** les agents auxiliaires (m) sont des colorants, des agents tensioactifs, des agents opacifiants, des agents de matage, s'agissant par exemple de dioxyde de titane ou d'oxyde de zinc, des agents plastifiants, des adsorbants/absorbant d'hydrogène, des substances radio-opaques ou de résines MQ organosiliciques pourvues de groupes Si-vinyle, Si-OR, Si-éthynyle et/ou SiH ou des composés ou des tampons et des matières destinés à ajuster le pH.

19. Mélanges pouvant être obtenus en mélangeant les composants A et B des revendications 2 à 18.

20. Mélanges selon la revendication 19, **caractérisés en ce que**, dans une première étape, le mélange réalisé à partir des composants change des consistance pour passer, pendant le processus de mélange et ensuite, d'un état relativement liquide et approprié au malaxage mécanique à un état plus visqueux et plastique garantissant une forte pression d'appui lors de la réalisation de l'empreinte dentaire dans un port-empreinte, la deuxième étape étant le durcissement du matériau qui atteindra ainsi sa forme finale élastique.

21. Mélanges selon la revendication 19, **caractérisés en ce que** lesdits mélanges ont achevé le processus de durcissement.

22. Mélanges selon la revendication 19, **caractérisés en ce qu'**au début du processus de mélange, les mélanges présentent un premier état lors duquel leur consistance est appropriée au malaxage mécanique et se **caractérise par** des valeurs de > 26 mm, de préférence de > 30 mm (selon ISO 4823), lesdits mélanges passant ensuite, en raison de réactions de condensation de groupes SiOR et/ou en raison de réactions d'hydrosilylation entre des groupes alcynyle et des groupes SiH, à un deuxième état plus visqueux avec une consistance de < 35 mm, de préférence de < 30 mm (selon ISO 4823), cette dernière consistance étant maintenue durant au moins 15 sec, lesdits mélanges atteignant ensuite, en raison d'une réaction d'hydrosilylation de groupes alcényle avec des groupes SiH, un troisième état solide mais élastique, une fois le processus de durcissement achevé.

23. Utilisation d'un système à plusieurs composants selon l'une des revendications 1 à 18 pour réaliser une empreinte d'un objet dont la forme doit être reproduite, le matériau d'empreinte étant préparé en mélangeant les composants, le matériau d'empreinte se trouvant dans un premier état lors qu'il est extrait d'un récipient pour ensuite adopter un deuxième état **caractérisé par** une viscosité accrue du matériau d'empreinte et permettant de réaliser l'empreinte de l'objet dont la forme doit être reproduite, le matériau d'empreinte passant finalement à un troisième état solide qui permet de conserver le résultat de la prise d'empreinte, ledit deuxième état étant atteint grâce à des réactions d'hydrosilylation différées des unités structurelles alcynyle et alcényle avec des composés pourvus de groupes Si-H et/ou grâce à la réaction d'addition différée (entre groupes alcényle et SiH) et à la réaction de condensation (de groupes SiOR avec des catalyseurs de condensation).

24. Utilisation selon la revendication 23, **caractérisée en ce qu'**au début du processus de mélange, la consistance du matériau d'empreinte dans son premier état correspond à des valeurs de > 26 mm, de préférence de > 30 mm (selon ISO 4823), le matériau d'empreinte dans son premier état étant approprié au malaxage, et que la consistance du matériau d'empreinte dans son deuxième état correspond à des valeurs de < 35 mm, de préférence de < 30 mm (selon ISO 4823), le matériau d'empreinte étant alors plus visqueux que dans son premier état, et que le matériau d'empreinte conserve ce deuxième état jusqu'à la fin de durée totale de l'application, pendant une durée d'au moins 15 sec.

25. Mélanges selon la revendication 19, **caractérisés en ce qu'**au début du processus de mélange, les mélanges présentent un premier état lors duquel leur consistance est appropriée au malaxage mécanique et se **caractérise par** des valeurs de > 26 mm, de préférence de > 30 mm (selon ISO 4823), lesdits mélanges passant ensuite, en raison d'une réaction d'hydrosilylation entre groupes alcényle et groupes SiH, à un deuxième état plus visqueux avec une consistance de < 35 mm, de préférence de < 30 mm (selon ISO 4823), cette dernière consistance étant maintenue durant au moins 15 sec, lesdits mélanges atteignant ensuite, en raison de réactions de condensation de groupes SiOR et/ou en raison de réactions d'hydrosilylation entre des groupes alcynyle et des groupes SiH, un troisième état solide mais élastique.
